# EUROPÄISCHE PATENTSCHRIFT

(11) **EP 3 672 688 B1**
(45) Veröffentlichungstag und Bekanntmachung des Hinweises auf die Patenterteilung: **01.03.2023**
(21) Anmeldenummer: 18755715.2
(22) Anmeldetag: 20.07.2018
(51) Int. Cl.: A61N 5/10

(54) **VERFAHREN ZUM BEREITSTELLEN VON ERGEBNISDATEN, WELCHE GEEIGNET FÜR EINEN EINSATZ IN EINER PLANUNG EINER BESTRAHLUNG EINES PATIENTEN SIND**
METHOD FOR PROVIDING RESULT DATA WHICH IS SUITABLE FOR USE IN PLANNING THE IRRADIATION OF A PATIENT
PROCÉDÉ PERMETTANT DE FOURNIR DES DONNÉES DE RÉSULTAT APPROPRIÉES POUR ÊTRE UTILISÉES DANS UNE PLANIFICATION D'UNE IRRADIATION D'UN PATIENT

(30) Priorität: 23.08.2017 US 201762549175 P
(43) Veröffentlichungstag der Anmeldung: 01.07.2020
(73) Patentinhaber: Siemens Healthcare GmbH, 91052 Erlangen (DE)
(72) Erfinder: AMIES, Christopher Jude, Sykesville, MD 21784 (US); HOFMANN, Christian, 91052 Erlangen (DE); HÖLZER, Philipp, Baltimore, MD 21201 (US); KARTMANN, René, 90491 Nürnberg (DE); KELLER-REICHENBECHER, Mark-Aleksi, 69207 Sandhausen (DE); KREISLER, Björn, 91353 Hausen (DE); RITTER, André, 91077 Neunkirchen am Brand (DE)
(86) Internationale Anmeldenummer: PCT/EP2018/069777
(87) Internationale Veröffentlichungsnummer: WO 2019/037977

(56) Entgegenhaltungen:
- EP-A2- 3 238 780
- DE-A1-102005 059 210
- DE-A1-102006 026 945

## Beschreibung

Die Erfindung betrifft ein Verfahren zum Bereitstellen von Ergebnisdaten, welche geeignet für einen Einsatz in einer Planung einer Bestrahlung eines Patienten sind, eine Recheneinheit, ein CT-Gerät und ein Computerprogrammprodukt.

Bei einer Strahlentherapie wird ein Zielvolumen, beispielsweise ein Tumor, eines Patienten mit ionisierender Strahlung bestrahlt. Hierbei ist eine externe Strahlentherapie, welche eine Bestrahlung eines Körpers des Patienten von außerhalb des Körpers umfasst, bekannt. Ebenfalls ist eine interne Strahlentherapie, auch Brachytherapie genannt, bekannt. Bei einer Brachytherapie werden Strahlenquellen, welche radioaktive Substanzen umfassen, in einen Körper des Patienten eingebracht, um lokal im Körper des Patienten das Tumorgewebe im Zielvolumen zu schädigen oder zu vernichten.

Grundsätzlich ist es eine Herausforderung bei der Bestrahlung des Patienten sicher zu stellen, dass eine derart ausreichende Strahlendosis einem Zielvolumen zugeführt wird, so dass das im Zielvolumen enthaltene Tumorgewebe zerstört wird. Gleichzeitig sollen das Zielvolumen umgebene Risikoorgane, welche zumindest ein Risikovolumen bilden, möglichst geschont werden. Damit ist eine hohe Genauigkeit bei der Planung und/oder Vorbereitung der Bestrahlung von hoher Bedeutung.

Es ist bekannt eine Strahlentherapie bzw. eine Bestrahlung eines Patienten mittels einer Bildgebung zu planen und/oder vorzubereiten. Hierzu wird üblicherweise ein Bestrahlungsplan mit Hilfe von medizinischen Messdaten des Patienten erstellt, die mit einem dreidimensionalen bildgebenden Verfahren erstellt wurden. Üblicherweise werden hierfür mittels eines Computertomographiegeräts (CT-Geräts) akquirierte Computertomographie-Messdaten (CT-Messdaten) eingesetzt. Anhand der Computertomographie-Messdaten kann einerseits das Zielvolumen der Bestrahlung festgelegt werden, und andererseits das umliegende Risikovolumen lokalisiert werden. Darüber hinaus bilden die Intensitätswerte der CT-Messdaten (gemessen in sogenannten "Hounsfield Units") in guter Näherung eine Elektronendichte am korrespondierenden Ort im Körper des Patienten ab, da die Intensitätswerte auf einer Absorption der Röntgenstrahlung an den zugehörigen Orten beruhen. Derart können die CT-Messdaten besonders einfach für die Planung der Bestrahlung in eine Elektronendichtekarte umgerechnet werden. Da bei einer Bestrahlung die Intensität der Wechselwirkung der Strahlung mit der Elektronendichte im Körper korreliert, kann aus den CT-Messdaten vergleichsweise einfach die Schwächung der Strahlung beim Durchtritt durch den Körper des Patienten errechnet werden. CT-Messdaten weisen weiterhin nur eine geringe geometrische Verzerrung auf und ermöglichen somit eine geeignete Definition einer Referenzgeometrie für die Planung der Bestrahlung und das Durchführen der Bestrahlung. Aufgrund dieser Eigenschaft wurden CT-Messdaten bisher bei der Planung der Bestrahlung bevorzugt eingesetzt.

In jüngster Zeit werden zur Verwendung in CT-Geräten neben den bisher verwendeten konventionellen Detektortypen mit integrierenden Detektorelementen solche quantenzählenden Röntgendetektoren vorgeschlagen. So ist beispielsweise aus der EP 1489969 B1 ein CT-Gerät bekannt, dessen Detektoren empfangene Röntgenstrahlung hinsichtlich der Anzahl an Röntgenquanten, deren Quantenenergie einen vorgegebenen Schwellwert überschreitet, erfassen kann. In der DE 10 2007 034 982 B4 ist ein Verfahren zum Betreiben eines quantenzählenden Röntgendetektors beschrieben. Aus der DE 10 2009 032 252 B3 ist ein Verfahren zur Erzeugung von Energie-selektiven Röntgenbildern eines Messfelds bekannt.

Ein quantenzählender Röntgendetektor, auch direktkonvertierender Röntgendetektor oder photonenzählender Röntgendetektor genannt, ermöglicht eine direkte Umwandlung von einem hochenergetischen Photon in Elektron-Loch Paare, wenn das hochenergetische Photon auf ein Halbleitermaterial des quantenzählenden Röntgendetektors trifft. Die im Halbleitermaterial generierten Elektronen können anschließend in einem integrierten Schaltkreis in einen elektrischen Signalpuls umgewandelt werden. Eine Pulshöhe des Signalpulses kann dabei mit einer Energie des hochenergetischen Photons korrelieren. Über eine Festlegung von geeigneten Energieschwellen können somit einzelne detektierte hochenergetische Photonen in verschiedenen Energiebändern gezählt werden.

Im Bereich der Computertomographie ermöglicht die Anwendung von quantenzählenden Röntgendetektoren verschiedene Vorteile. So können die mittels des CT-Geräts akquirierten CT-Messdaten eine intrinsische spektrale Sensitivität aufweisen, da die Energie der detektierten Photonen, wie im vorhergehenden Abschnitt erläutert, direkt detektiert werden kann. Weiterhin weisen die mittels des CT-Geräts akquirierten CT-Messdaten eine intrinsische hohe Auflösung auf, da die Detektorelemente des quantenzählenden Röntgendetektors typischerweise kleiner sind als die Detektorelemente von herkömmlichen Röntgendetektoren. Weiterhin weisen quantenzählende Röntgendetektoren üblicherweise eine gute Performance bei geringer Signalintensität auf, da Elektronikrauschen typischerweise nahezu komplett unterdrückt wird, da das Elektronikrauschen unter der ersten gesetzten Energieschwelle liegt. Weiterhin ermöglicht das Zählen von einzelnen Photonen eine Verringerung einer inhärenten Energiegewichtung des quantenzählenden Röntgendetektors.

Die DE 10 2005 059210 A1 beschreibt eine radiotherapeutische Vorrichtung mit einer radiotherapeutischen Bestrahlungseinheit mit einer Strahlungsquelle zur Erzeugung von radiotherapeutischer Strahlung und einer Strahlführungs- und/oder Strahlformungseinrichtung, um die radiotherapeutische Strahlung definiert auf einen bestimmten Bestrahlungsbereich zu richten. Zusätzlich besitzt die radiotherapeutische Vorrichtung eine Bildgebungseinheit welche eine Radionuklid-Emissions-Tomographie-Aufnahmeeinheit umfasst.

Die nachveröffentlichte EP 3 238 780 A2 beschreibt ein Verfahren zur Unterstützung einer Planung einer Bestrahlung eines Patienten, bei welchem Magnetresonanz-Bilddaten zu Computertomographie-Bilddaten eines Patienten zugeordnet werden, wobei die Magnetresonanz-Bilddaten von einem von dem Patienten verschiedenen Untersuchungsobjekt akquiriert worden sind, und bei welchem die Computertomographie-Bilddaten zusammen mit den den Computertomographie-Bilddaten zugeordneten Magnetresonanz-Bilddaten zur Unterstützung der Planung der Bestrahlung des Patienten bereitgestellt werden.

Die DE 10 2006 026945 A1 beschreibt ein computertomographisches Bildaufnahmeverfahren, bei welchem unter Bestrahlung eines Untersuchungsobjekts mit polychromatischer Röntgenstrahlung entlang mehrerer Projektionsvektoren Projektionsbilder der durch das Untersuchungsobjekt transmittierten Röntgenstrahlung aufgenommen werden und mittels eines Rekonstruktionsalgorithmus anhand der Projektionsbilder ein ortsabhängiger Schwächungskoeffizient des Untersuchungsobjekts berechnet wird.

Der Erfindung liegt die Aufgabe zu Grunde, ein Verfahren zum Bereitstellen von Ergebnisdaten, welche besonders geeignet für einen Einsatz in einer Planung einer Bestrahlung eines Patienten sind, anzugeben. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zum Bereitstellen von Ergebnisdaten, welche geeignet für einen Einsatz in einer Planung einer Bestrahlung eines Patienten sind, umfasst folgende Verfahrensschritte:
- Erfassen von CT-Messdaten des Patienten, welche mittels eines CT-Geräts, welches einen quantenzählenden Röntgendetektor aufweist, akquiriert worden sind,
- Weiterverarbeitung der CT-Messdaten, wobei bei der Weiterverarbeitung der CT-Messdaten ein spezifischer Informationsgehalt der CT-Messdaten, welcher sich aus der Verwendung des quantenzählenden Röntgendetektors bei der Akquisition der CT-Messdaten ergibt, berücksichtigt wird, wobei bei der Weiterverarbeitung der CT-Messdaten Ergebnisdaten erzeugt werden, welche geeignet für den Einsatz in der Planung der Bestrahlung des Patienten sind,
- Bereitstellen der Ergebnisdaten an eine Schnittstelle, so dass die Ergebnisdaten für die Planung der Bestrahlung des Patienten verwendet werden können.

Generell kann der Einsatz eines quantenzählenden Röntgendetektors eine Akquisition von CT-Messdaten ermöglichen, welche besonders geeignet für den Einsatz in der Planung der Bestrahlung des Patienten sind. Weiterhin können mittels einer geeigneten Weiterverarbeitung der akquirierten CT-Messdaten, welche besonders vorteilhaft auf den spezifischen Informationsgehalt der CT-Messdaten abgestimmt ist, Ergebnisdaten erzeugt werden, welche besonders geeignet für den Einsatz in der Planung der Bestrahlung des Patienten sind.

An CT-Messdaten und/oder an aus den CT-Messdaten weiterverarbeitete Ergebnisdaten können verschiedene Anforderungen gestellt werden, damit diese Daten besonders geeignet für den Einsatz in der Planung der Bestrahlung des Patienten sind:
Es kann vorteilhaft sein, dass die CT-Messdaten eine hohe Auflösung und/oder einen hohen Weichteilkontrast (mit oder ohne Kontrastmittel) aufweisen, damit in der Weiterverarbeitung der CT-Messdaten ein Zielvolumen und/oder Risikovolumen für die Bestrahlung so exakt wie möglich identifiziert und abgegrenzt werden kann. Diese Voraussetzung kann auch bei dem Auftreten von einer Bewegung von Organen des Patienten, beispielsweise in der Thorax-, Abdomen oder Pelvisregion, weiterhin entscheidend sein. Die CT-Messdaten sollten insbesondere dabei geeignet für die Identifikation von Organgrenzen der Organe sein, welche beispielsweise mittels einer manuellen, semi-automatischen oder automatischen Konturierung und/oder Segmentierung erfolgt. So kann beispielsweise eine Abgrenzung zwischen Muskelgewebe und Fettgewebe oder zwischen grauer und weißer Hirnsubstanz bei der Weiterverarbeitung der CT-Messdaten nötig sein. Weiterhin können funktionelle CT-Messdaten, wie beispielsweise Perfusions-Messdaten oder Ventilations-Messdaten, bei der Konturierung des Zielvolumens und/oder Risikovolumens, vorteilhaft eingesetzt werden.

Für die Dosisberechnung bei der Planung der Bestrahlung, insbesondere bei der Partikeltherapie mit Protonen oder Kohlenstoffionen, ist es insbesondere wichtig, dass in der Weiterverarbeitung der CT-Messdaten quantitative Materialkoeffizienten, wie beispielsweise eine Elektronendichte und/oder eine Massendichte und/oder eine effektive Kernladungszahl und/oder ein Bremsvermögen (Stopping Power), zuverlässig aus den CT-Messdaten abgeleitet werden können. Die quantitativen Materialkoeffizienten können dann als eine Basis für eine Dosisberechnung für die Planung der Bestrahlung des Patienten dienen. Insbesondere bei einer Partikeltherapie, einer Bestrahlung des Patienten mit ionisierten Teilchen, ist es vorteilhaft, dass das Bremsvermögen und/oder die korrespondierende Wasser-äquivalente Pfadlänge (water equivalent path length, WEPL) basierend auf den CT-Messdaten berechnet wird. Auch ermöglicht die Verwendung des quantenzählenden Röntgendetektors eine Akquisition eines Normalisierungs-Bilds für jede CT-Messdatenakquisition, so dass eine Berechnung des quantitativen Materialkoeffizienten für die Konturierung oder die Dosisberechnung normalisiert durchgeführt werden kann.

Bei der Vorbereitung der Bestrahlung oder während der Bestrahlung können CT-Messdaten, insbesondere Cone-Beam CT-Messdaten, welche mittels eines quantenzählenden flat-panel Detektors akquiriert werden, besonders vorteilhaft zum Verfolgen (Tracking) des Zielvolumens und/oder des Risikovolumens verwendet werden. Die mittels des quantenzählenden Röntgendetektors akquirierten CT-Messdaten können dann besonders vorteilhaft dazu eingesetzt werden, um die Positionierung des Patienten, insbesondere des Zielvolumens, für die Bestrahlung zu verifizieren. Ebenfalls ist die Durchführung einer Online-Dosimetrie denkbar.

Schließlich können CT-Messdaten besonders vorteilhaft zum Beobachten (Monitoring) eines Behandlungsverlaufs, beispielsweise bei Follow-Up Untersuchungen, eingesetzt werden. So kann mittels der CT-Messdaten eine Reaktion des Gewebes auf die Bestrahlung, beispielsweise in Form einer Entzündung, einer Tumorregression oder Tumorprogression, verfolgt werden. Dementsprechend können anschließend Bestrahlungsparameter für die Bestrahlung des Patienten im Sinne der adaptiven Bestrahlung geeignet angepasst werden. Auch kann ein Ansprechen des Patienten auf die Bestrahlung mittels einer Evaluierung von Tumorparametern, wie beispielsweise Tumorgröße und/oder Tumorvolumen und/oder Angiogenese, und mittels eines Vergleichs dieser Tumorparameter zu früheren Messungen untersucht werden. Die mittels des quantenzählenden Röntgendetektors akquirierten CT-Messdaten können in Kombination mit einer Erkennung von bestimmten Bildmerkmalen, beispielsweise Texturparametern, im Zielvolumen und/oder Risikovolumen besonders geeignet verwendet werden, um das Treffen von Behandlungsentscheidungen für den Patienten zu unterstützen oder zu ermöglichen.

Im Folgenden soll beschrieben werden, wie mittels des Einsatzes des quantenzählenden Röntgendetektors CT-Messdaten akquiriert werden können, welche die genannten Anforderungen zumindest zum Teil besonders geeignet erfüllen können. Auch soll auf eine besonders geeignete Weiterverarbeitung der CT-Messdaten für den Einsatz in der Planung der Bestrahlung des Patienten eingegangen werden.

Das CT-Gerät kann ein typisches CT-Gerät sein, welches zur Akquisition von Planungsbilddaten zur Planung der Bestrahlung des Patienten eingesetzt wird. In diesem Fall werden die CT-Messdaten üblicherweise zeitlich vor einem Start der ersten Bestrahlung des Patienten akquiriert. Die Bestrahlung des Patienten findet dann üblicherweise zumindest einen Tag nach der Akquisition der CT-Messdaten statt. Weiterhin ist in diesem Fall typischerweise das CT-Gerät in einem Untersuchungsraum positioniert, welcher von einem Behandlungsraum, in welchem die Bestrahlung des Patienten mittels des Strahlentherapiegeräts stattfindet, räumlich separiert ist.

Es ist allerdings auch denkbar, dass das CT-Gerät in dem Behandlungsraum, insbesondere einem Strahlenbunker, in welche die Bestrahlung des Patienten mittel des Strahlentherapiegeräts stattfindet, zusammen mit dem Strahlentherapiegerät installiert ist. Das CT-Gerät kann dann einen Teil des Strahlentherapiegeräts darstellen oder separat vom Strahlentherapiegerät installiert sein. Die Patientenlagerungsvorrichtung kann zwischen dem Strahlentherapiegerät und dem CT-Gerät hin und her fahren. In dem in diesem Absatz beschriebenen Fall können die akquirierten CT-Messdaten besonders vorteilhaft für eine adaptive Planung der Bestrahlung des Patienten, insbesondere für eine Anpassung eines bereits existierenden Bestrahlungsplans, und/oder für eine Verifikation einer Positionierung des Patienten für die Bestrahlung eingesetzt werden.

Es ist in speziellen Fällen auch denkbar, dass das CT-Gerät einen C-Bogen aufweist, wobei die Röntgenquelle und der zumindest eine quantenzählende Röntgendetektor an gegenüberliegenden Enden des C-Bogens befestigt sind.

CT-Messdaten sind insbesondere mittels des CT-Geräts akquirierte Projektionsdaten oder aus den Projektionsdaten rekonstruierte Bilddaten. Das Erfassen der CT-Messdaten kann ein Laden der mittels des quantenzählenden Röntgendetektors akquirierten CT-Messdaten aus einer Datenbank umfassen. Das Erfassen der CT-Messdaten kann auch die Akquisition der CT-Messdaten mittels des quantenzählenden Röntgendetektors umfassen.

Die CT-Messdaten, welche mittels des quantenzählenden Röntgendetektors akquiriert worden sind, können auch Cone-Beam CT-Messdaten (CBCT-Messdaten) sein. Die CBCT-Messdaten werden insbesondere dann akquiriert, wenn der Patient bereits für die Bestrahlung mittels des Strahlentherapiegeräts auf einer Patientenlagerungsvorrichtung des Strahlentherapiegeräts positioniert ist. Die CBCT-Messdaten werden insbesondere dann akquiriert, wenn der Patient derart positioniert ist, dass er für die Bestrahlung mittels des Strahlentherapiegeräts nicht mehr umgelagert werden muss. Die Akquisition der CBCT-Messdaten erfolgt dann insbesondere zeitlich unmittelbar vor dem Start der Bestrahlung des Patienten oder wenn die Bestrahlung des Patienten bereits begonnen hat. In diesem Fall weist insbesondere eine Bildgebungsvorrichtung des Strahlentherapiegeräts den quantenzählenden Röntgendetektor auf. Die derart akquirierten CBCT-Messdaten können in diesem Fall für unterschiedliche vorteilhafte Anwendungen im Rahmen der Planung bzw. Vorbereitung der Bestrahlung des Patienten akquiriert werden:
- für eine adaptive Planung der Bestrahlung des Patienten, insbesondere für eine Anpassung eines bereits existierenden Bestrahlungsplans,
- für eine online Dosimetrie,
- für eine Verifikation einer Positionierung des Patienten für die Bestrahlung,
- für eine Verfolgung des Zielvolumens und/oder Risikovolumens während der Durchführung der Bestrahlung des Patienten, insbesondere während einer Atembewegung des Patienten.

Die CBCT-Messdaten können für einen oder eine beliebige Kombination der genannten Anwendungen eingesetzt werden.

Im Falle des Einsatzes der mittels des quantenzählenden Röntgendetektors akquirierten CBCT-Messdaten für die online Dosimetrie ist eine Erhöhung der Genauigkeit der dem Patienten zugefügten Strahlendosis denkbar. Insbesondere ist eine direkte Berechnung der Strahlendosis aus den CBCT-Messdaten denkbar. Derart kann auf modellbasierte Verfahren zur Dosisberechnung verzichtet werden. Der quantenzählende Röntgendetektor kann vorteilhafterweise kleine Unterschiede in der Höhe der Strahlendosis, welche unterschiedlichen Organen des Patienten, beispielsweise der Lunge oder Knochen, zugefügt worden ist, erkennen.

Die Weiterverarbeitung der CT-Messdaten erfolgt insbesondere mittels eines Weiterverarbeitungsalgorithmus, wobei die CT-Messdaten als Eingangsparameter in den Weiterverarbeitungsalgorithmus eingehen. Der Ausgangsparameter des Weiterverarbeitungsalgorithmus werden insbesondere die Ergebnisdaten erzeugt. Die Weiterverarbeitung der CT-Messdaten umfasst insbesondere einen solchen zumindest einen Weiterverarbeitungsschritt, dass aus den CT-Messdaten Ergebnisdaten, welche geeignet für den Einsatz in der Planung der Bestrahlung des Patienten sind, erzeugt werden. Derart kann die Weiterverarbeitung der CT-Messdaten eine Konvertierung der CT-Messdaten, beispielsweise in eine ortsaufgelöste Verteilung eines Materialkoeffizienten, umfassen. Auch kann die Weiterverarbeitung der CT-Messdaten eine Konturierung und/oder Segmentierung einer Organstruktur, insbesondere des Zielvolumens und/oder Risikovolumens in den CT-Messdaten, umfassen. Es sind selbstverständlich weitere Möglichkeiten der Weiterverarbeitung der CT-Messdaten denkbar. Verschiedene Möglichkeiten zur Weiterverarbeitung der CT-Messdaten sind in der Beschreibung zu den Ausführungsformen beschrieben.

Der spezifische Informationsgehalt der CT-Messdaten ergibt sich aus der Verwendung des quantenzählenden Röntgendetektors bei der Akquisition der CT-Messdaten. So kann der spezifische Informationsgehalt der CT-Messdaten beispielsweise eine spektrale Auflösung der CT-Messdaten sein, welche sich aus der inhärenten Energiesensitivität des quantenzählenden Röntgendetektors ergibt. Auch ist es denkbar, dass der spezifische Informationsgehalt eine besonders hohe räumliche Auflösung der CT-Messdaten ist, welche sich aus der Verwendung des quantenzählenden Röntgendetektors bei der Akquisition der CT-Messdaten ergibt. Grundsätzlich kann die Verwendung des quantenzählenden Röntgendetektors auch die Weiterverarbeitung der CT-Messdaten vereinfachen, so dass besonders einfach geeignete Ergebnisdaten aus den CT-Messdaten erzeugt werden. Dann liegt der spezifische Informationsgehalt der CT-Messdaten in der besonderen Eignung der CT-Messdaten für die Weiterverarbeitung zu den Ergebnisdaten, welche geeignet für den Einsatz in der Planung der Bestrahlung des Patienten sind. Der spezifische Informationsgehalt der CT-Messdaten kann sich auch durch eine besonders geschickte Akquisition der CT-Messdaten ergeben, welche erst durch die Verwendung des quantenzählenden Röntgendetektors möglich ist.

Der spezifische Informationsgehalt der CT-Messdaten wird insbesondere derart bei der Weiterverarbeitung der CT-Messdaten berücksichtigt, dass eine besonders einfache und/oder effiziente Weiterverarbeitung der CT-Messdaten zur Erzeugung der Ergebnisdaten ermöglicht wird. Auch kann der spezifische Informationsgehalt der CT-Messdaten erst die geeignete Weiterverarbeitung der CT-Messdaten überhaupt ermöglichen.

So können mittels der Weiterverarbeitung der CT-Messdaten Ergebnisdaten erzeugt werden, welche besonders geeignet für den Einsatz in der Planung der Bestrahlung des Patienten sind. Die Ergebnisdaten können dabei unterschiedliche Gestalt haben, beispielsweise in Form einer ortsaufgelösten Verteilung eines Materialkoeffizienten oder einer Kontur einer Organstruktur vorliegen. Grundsätzlich stellen die Ergebnisdaten geeignete Eingangsparameter für einen Bestrahlungsplanungsalgorithmus dar, mittels welchem ein Bestrahlungsplan für die Bestrahlung des Patienten erstellt werden kann. Selbstverständlich können neben den Ergebnisdaten noch andere Daten als Eingangsparameter in den Bestrahlungsplanungsalgorithmus eingehen. Die mittels des quantenzählenden Röntgendetektors akquirierten CT-Messdaten können beispielsweise kombiniert mit Messdaten von anderen Bildgebungsmodalitäten, beispielsweise MR-Messdaten oder PET-Messdaten, in die Planung der Bestrahlung des Patienten eingehen.

Der Bestrahlungsplanungsalgorithmus kann insbesondere auf die Schnittstelle, insbesondere die an die Schnittstelle bereitgestellten Ergebnisdaten, zugreifen, um die tatsächliche Planung der Bestrahlung des Patienten durchzuführen. Die Planung der Bestrahlung des Patienten ist derart, wie in der folgenden Ausführungsform beschrieben, ein besonders vorteilhafter, insbesondere dem erfindungsgemäßen Verfahren nachgelagerter Schritt.

Eine Ausführungsform sieht vor, dass das Verfahren den folgenden zusätzlichen Verfahrensschritt umfasst: Durchführen der Planung der Bestrahlung des Patienten, wobei die Ergebnisdaten von der Schnittstelle abgerufen werden und bei der Planung der Bestrahlung des Patienten eingesetzt werden.

In diesem Fall greift der Bestrahlungsplanungsalgorithmus insbesondere auf die in der Schnittstelle hinterlegten Ergebnisdaten zu, um diese abzurufen und bei der Planung der Bestrahlung des Patienten einzusetzen. Beispielsweise können die Ergebnisdaten eine ortsaufgelöste Verteilung eines Materialkoeffizienten darstellen, welche als Eingangsparameter für eine Dosisberechnung Bestrahlungsplanungsalgorithmus verwendet werden kann. Gemäß einer weiteren Möglichkeit umfassen die Ergebnisdaten eine Konturierung eines Zielvolumens und/oder Risikovolumens, welche als Grundlage für die Planung der Bestrahlung verwendet werden kann. Die Ergebnisdaten können selbstverständlich auch auf andere, den Fachmann als sinnvoll erscheinende Weise in die Planung der Bestrahlung eingehen.

Eine Ausführungsform sieht vor, dass das Erfassen der CT-Messdaten ein Erfassen von spektral-aufgelösten CT-Messdaten, welche sich aus der Akquisition mittels des quantenzählenden Röntgendetektors ergeben, umfasst, die Weiterverarbeitung der CT-Messdaten ein Berechnen einer ortsaufgelösten Verteilung eines quantitativen Materialkoeffizienten aus den spektral-aufgelösten CT-Messdaten umfasst und das Bereitstellen der Ergebnisdaten ein Bereitstellen der ortsaufgelösten Verteilung des quantitativen Materialkoeffizienten an die Schnittstelle umfasst.

Der quantitative Materialkoeffizient charakterisiert insbesondere eine physikalische Eigenschaft des Gewebes. Dementsprechend weist der quantitative Materialkoeffizient üblicherweise eine physikalische Einheit auf. Der quantitative Materialkoeffizient wird insbesondere für jeden Voxel der CT-Messdaten berechnet. Der quantitative Materialkoeffizient kann aus folgender Liste ausgewählt werden: eine Elektronendichte, eine Massendichte, eine effektive Kernladungszahl, ein linearer Abschwächungskoeffizient für eine bestimmte Energie oder für ein Spektrum aus mehreren Energien, ein Bremsvermögen (Stopping Power), eine Wasser-äquivalente Pfadlänge (water equivalent path length, WEPL), eine quantitative elementare Zusammensetzung des Gewebes. Selbstverständlich können auch mehrere quantitative Materialkoeffizienten, welche eine beliebige Auswahl aus dieser Liste darstellen, berechnet werden. Auch sind selbstverständlich weitere, dem Fachmann als sinnvoll erscheinende quantitative Materialkoeffizienten denkbar.

Die ortsaufgelöste Verteilung des quantitativen Materialkoeffizienten kann besonders geeignet aus den mittels des quantenzählenden Röntgendetektors akquirierten CT-Messdaten berechnet werden. Der Hauptgrund dafür ist insbesondere, dass die CT-Messdaten aufgrund der Akquisition mittels des quantenzählenden Röntgendetektors spektral-aufgelöst vorliegen können. Dies bedeutet insbesondere, dass bei der Akquisition der CT-Messdaten der quantenzählende Röntgendetektor und die Röntgenröhre des CT-Geräts geeignet konfiguriert ist, dass CT-Messdaten für zumindest zwei verschiedene Energiespektren akquiriert werden. Aufgrund der inhärenten Energieauflösung kann der quantitative Materialkoeffizient auch retrospektiv aus ursprünglich für einen anderen Zweck mittels des quantenzählenden Röntgendetektors akquirierten spektral-aufgelösten CT-Messdaten berechnet werden. Dieses Vorgehen ist in einem der folgenden Ausführungsformen noch genauer beschrieben.

Die Verwendung von spektral-aufgelösten CT-Messdaten bei der Berechnung des quantitativen Materialkoeffizienten ist gegenüber der Verwendung von konventionellen CT-Messdaten, welche lediglich bei einer einzelnen Energie bzw. einem einzelnen Energiespektrum vorliegen, vorteilhaft. Der quantitative Materialkoeffizient kann nämlich insbesondere direkt aus den spektral-aufgelösten CT-Messdaten berechnet werden. In die Berechnung der ortsaufgelösten Verteilung des quantitativen Materialkoeffizienten können dann insbesondere ausschließlich die spektral-aufgelösten CT-Messdaten eingehen.

Dagegen kann der quantitative Materialkoeffizient aus den konventionellen CT-Messdaten typischerweise nur über Umwege berechnet werden. Denn die Hounsfield-Werte (HU-Werte) der konventionellen CT-Messdaten können typischerweise nicht direkt für die Berechnung des quantitativen Materialkoeffizienten verwendet werden, da sie keine spektral-aufgelösten Energieinformationen umfassen, jedoch die Abschwächung in einem gegebenen Material üblicherweise eine Funktion der Energie ist. Damit aus konventionellen CT-Messdaten der quantitative Materialkoeffizient berechnet werden kann, ist demnach typischerweise eine Kalibrierungsmessung und ein Abspeichern einer Konversionsvorschrift in einer Look-Up Tabelle nötig. Daraus folgt, dass für die konventionelle CT-Akquisition üblicherweise Limitierungen durchgeführt werden müssen, beispielsweise die Röhrenspannung nicht verändert werden darf.

Diese Nachteile können durch die Verwendung des quantenzählenden Röntgendetektors bei der Akquisition der spektral-aufgelösten CT-Messdaten besonders geeignet adressiert werden, da aufgrund der Energieauflösung die spektral-aufgelösten CT-Messdaten vorteilhafterweise direkt in die Berechnung des quantitativen Materialkoeffizienten eingehen können. Derart kann vorteilhafterweise die Kalibrierungsmessung, welche üblicherweise für die konventionellen CT-Messdaten nötig ist, entfallen. Weiterhin können vorteilhafterweise Limitierungen bei der Akquisition der spektral-aufgelösten CT-Messdaten entfallen.

Auch ist vorteilhafterweise eine Qualität bzw. eine Genauigkeit des aus den spektral-aufgelösten CT-Messdaten rekonstruierten quantitativen Materialkoeffizienten höher als aus konventionellen CT-Messdaten. Gründe hierfür sind beispielsweise, dass die Abhängigkeit von der Kalibrierungsmessung und somit von bei der Akquisition gewählten Akquisitionsparametern entfallen kann. Auch kann eine Abhängigkeit von einer elementaren Zusammensetzung des Gewebes, welche die in der Kalibrierung ermittelte Konversionsvorschrift möglicherweise beeinflusst, entfallen.

Weiterhin können die spektral-aufgelösten CT-Messdaten mittels des quantenzählenden Röntgendetektors optimiert für zusätzliche Aufgaben, beispielsweise besonders optimiert auf eine anschließende Konturierung des Zielvolumens und/oder Risikovolumens, akquiriert werden. Beispielsweise kann ein besonders geeignetes Kontrast-zu-Rauschverhältnis (CNR) in den spektral-aufgelösten CT-Messdaten sichergestellt werden, so dass die spektral-aufgelösten CT-Messdaten neben der Berechnung des quantitativen Materialkoeffizienten auch besonders geeignet für die Konturierung sind. Es ist vorteilhafterweise nicht nötig im Vorfeld der Akquisition der spektral-aufgelösten CT-Messdaten festzulegen, welcher quantitative Materialkoeffizient aus den spektral-aufgelösten CT-Messdaten rekonstruiert werden soll. Es ist idealerweise nicht nötig, Akquisitionsparameter der Akquisition der spektral-aufgelösten CT-Messdaten anzupassen, damit der quantitative Materialkoeffizient aus den spektral-aufgelösten CT-Messdaten rekonstruiert werden kann. Weiterhin ist es denkbar, dass unterschiedliche quantitative Materialkoeffizienten aus den spektral-aufgelösten CT-Messdaten rekonstruiert werden.

Die ortsaufgelöste Verteilung des quantitativen Materialkoeffizienten wird insbesondere für einen Dosisberechnungsalgorithmus bereitgestellt. Selbstverständlich sind auch andere Anwendungen des quantitativen Materialkoeffizienten in Bezug auf die Planung der Bestrahlung des Patienten denkbar.

Für die Akquisition der spektral-aufgelösten CT-Messdaten wird vorteilhafterweise ein standardisiertes Akquisitionsprotokoll definiert. Damit kann vorteilhafterweise der quantitative Materialkoeffizient auf eine standardisierte Weise direkt aus den spektral-aufgelösten CT-Messdaten rekonstruiert werden. Das standardisierte Akquisitionsprotokoll umfasst beispielsweise standardisierte Energieschwellen, welche besonders geeignet für die Berechnung des quantitativen Materialkoeffizienten, beispielsweise der Elektronendichte, sind. Weiterhin kann die standardisierte Akquisition der spektral-aufgelösten CT-Messdaten eine Weiterverarbeitung der aus den spektral-aufgelösten CT-Messdaten erhaltenen ortsaufgelösten Verteilung eines quantitativen Materialkoeffizienten erleichtern. Beispielsweise ist eine standardisierte Berechnung von ortsaufgelösten Verteilungen von zwei verschiedenen quantitativen Materialkoeffizienten aus den spektral-aufgelösten CT-Messdaten besonders vorteilhaft. Diese zwei-Parameter Modelle können eine besonders geeignete Grundlage für die Planung der Bestrahlung des Patienten liefern. Dieses Vorgehen wird in den folgenden Ausführungsformen genauer beschrieben.

Der mittels der CT-Messdaten berechnete quantitative Materialkoeffizient kann auch geeignet für Radiomics-Studien bzw. Follow-Up-Studien eingesetzt werden. Es kann ein quantitativer Materialkoeffizient, welcher charakteristisch für einen bestimmten Körperbereich des Patienten ist, akquiriert werden, insbesondere mit einer in einem der folgenden Absätze noch genauer beschriebenen Anpassung des Detektorparameters. Dieser charakteristische quantitative Materialkoeffizient kann verwendet werden, um ein Merkmalsmuster (feature pattern) in einer Datenbank, beispielsweise mittels eines statistischen Verfahrens oder eines maschinellen Lernverfahrens, zu identifizieren. Der charakteristische quantitative Materialkoeffizient kann auch zur Klassifizierung eines Gewebes eingesetzt werden, beispielsweise um einen Erfolg der Bestrahlung des Gewebes oder um eine Toxizität der Bestrahlung bezüglich des Gewebes zu quantifizieren.

Grundsätzlich ermöglicht die Verwendung des quantenzählenden Röntgendetektors eine inhärente spektrale Sensitivität der akquirierten CT-Messdaten. Derart können, wie in einer der folgenden Ausführungsformen noch genauer beschrieben, zwei quantitative Materialkoeffizienten von jeglichem zwei-Parameter-Modell berechnet werden. Alternativ oder zusätzlich ist auch, wie in einer der folgenden Ausführungsformen noch genauer beschrieben, eine Quantifizierung bezüglich zwei linear-unabhängiger Basismaterialien möglich. Dies führt vorteilhafterweise zu der Möglichkeit, dass unabhängig von einer Ausbildung des verwendeten CT-Geräts und von verwendeten Akquisitionsparametern, ein Satz von absoluten quantitativen Materialkoeffizienten für die Weiterverarbeitung im Rahmen der Planung der Bestrahlung des Patienten verwendet werden kann. Weiterverarbeitungsschritte im Rahmen dieser Weiterverarbeitung, wie beispielsweise die automatische Konturierung des Zielvolumens und/oder Risikovolumens oder bezüglich eines Therapiemonitorings, können demnach standardisiert durchgeführt werden.

Schließlich ist zu erwähnen, dass basierend auf der berechneten ortsaufgelösten Verteilung des quantitativen Materialkoeffizienten, insbesondere der Elektronendichte, Streu-Charakteristika (scatter characteristics) des Patienten definiert werden können. Derart kann die ortsaufgelöste Verteilung des Materialkoeffizienten auch als Eingangsparameter für einen Streu-Korrektur Algorithmus der CT-Messdaten verwendet werden.

Eine Ausführungsform sieht vor, dass die Weiterverarbeitung der CT-Messdaten ein Berechnen einer ersten ortsaufgelösten Verteilung eines ersten quantitativen Materialkoeffizienten und einer zweiten ortsaufgelösten Verteilung eines zweiten quantitativen Materialkoeffizienten aus den spektral-aufgelösten CT-Messdaten umfasst.

Sowohl die erste ortsaufgelöste Verteilung als auch die zweite ortsaufgelöste Verteilung können in diesem Fall die Ergebnisdaten bilden. Der erste quantitative Materialkoeffizient und der zweite quantitative Materialkoeffizient sind insbesondere unterschiedlich ausgebildet und beschreiben insbesondere unterschiedliche physikalische Gewebeeigenschaften. Vorteilhafterweise können auf Grundlage eines einzelnen Satzes an spektral-aufgelösten Messdaten sowohl die die erste ortsaufgelöste Verteilung als auch die zweite ortsaufgelöste Verteilung berechnet werden.

Eine Ausführungsform sieht vor, dass das Bereitstellen der Ergebnisdaten ein Bereitstellen der ersten ortsaufgelösten Verteilung des ersten quantitativen Materialkoeffizienten für einen ersten Dosisberechnungsalgorithmus und ein Bereitstellen der zweiten ortsaufgelösten Verteilung des zweiten quantitativen Materialkoeffizienten für einen zweiten Dosisberechnungsalgorithmus umfasst.

Der erste Dosisberechnungsalgorithmus verwendet dann insbesondere die erste ortsaufgelöste Verteilung des ersten quantitativen Materialkoeffizienten als Eingangsparameter für die Dosisberechnung. Im Rahmen der Planung der Bestrahlung des Patienten wird also insbesondere mittels des ersten Dosisberechnungsalgorithmus eine erste Dosisverteilung unter Berücksichtigung der ersten ortsaufgelösten Verteilung des ersten quantitativen Materialkoeffizienten berechnet. Gleichermaßen verwendet der zweite Dosisberechnungsalgorithmus insbesondere die zweite ortsaufgelöste Verteilung des zweiten quantitativen Materialkoeffizienten als Eingangsparameter für die Dosisberechnung. Im Rahmen der Planung der Bestrahlung des Patienten wird also insbesondere mittels des zweiten Dosisberechnungsalgorithmus eine zweite Dosisverteilung unter Berücksichtigung der zweiten ortsaufgelösten Verteilung des zweiten quantitativen Materialkoeffizienten berechnet.

Der erste Dosisberechnungsalgorithmus und der zweite Dosisberechnungsalgorithmus sind insbesondere unterschiedlich ausgebildet. Gerade unter Berücksichtigung der verschiedenen ortsaufgelösten Verteilungen des quantitativen Materialkoeffizienten können derart der erste Dosisberechnungsalgorithmus und der zweite Dosisberechnungsalgorithmus unterschiedliche Dosisverteilungen als Berechnungsergebnis aufweisen.

Eine Erhöhung der Zuverlässigkeit von Algorithmen zur Weiterverarbeitung der CT-Messdaten ist denkbar, wenn der erste quantitative Materialkoeffizient und der zweite quantitative Materialkoeffizient geeignet gewählt werden. Gemäß einer Ausführungsform ist der erste quantitative Materialkoeffizient eine Elektronendichte und der zweite quantitative Materialkoeffizient ist eine effektive Kernladungszahl. Gemäß einer weiteren Ausführungsform basieren der erste quantitative Materialkoeffizient und der zweite quantitative Materialkoeffizient auf zwei voneinander linear-unabhängigen Basismaterialien. Ein Beispiel für zwei voneinander linear-unabhängige Basismaterialien sind Wasser und Kalzium. Ein weiteres Beispiel ist Wasser und Iod.

Eine Ausführungsform sieht vor, dass die Weiterverarbeitung der CT-Messdaten eine getrennte Weiterverarbeitung der ersten ortsaufgelösten Verteilung des ersten quantitativen Materialkoeffizienten und der zweiten ortsaufgelösten Verteilung des zweiten quantitativen Materialkoeffizienten und eine Erzeugung der Ergebnisdaten mittels einer Zusammenführung der bei der getrennten Weiterverarbeitung erzeugten Teil-Ergebnisdaten umfasst.

Der erste quantitative Materialkoeffizient wird dabei insbesondere unabhängig vom zweiten quantitativen Materialkoeffizienten bestimmt. Durch die getrennte Berücksichtigung von zwei verschiedenen quantitativen Materialkoeffizienten ist eine weitere Erhöhung der Zuverlässigkeit von Algorithmen zur Weiterverarbeitung der CT-Messdaten denkbar. Die Algorithmen können beispielsweise zur Konturierung des Zielvolumens und/oder Risikovolumens dienen. Die Algorithmen können auch zur Analyse von Bildeigenschaften in manuell oder semiautomatisch generierten konturierten Bildvolumina dienen.

Eine Ausführungsform sieht vor, dass das Erfassen der CT-Messdaten ein Laden von zuvor akquirierten spektral-aufgelösten CT-Messdaten aus einer Datenbank zur Berechnung der ortsaufgelösten Verteilung des quantitativen Materialkoeffizienten umfasst.

Aufgrund der inhärenten Energieauflösung kann der quantitative Materialkoeffizient auch retrospektiv aus ursprünglich für einen anderen Zweck mittels des quantenzählenden Röntgendetektors akquirierten spektral-aufgelösten CT-Messdaten berechnet werden. Dennoch kann nachträglich aus den spektral-aufgelösten CT-Messdaten die ortsaufgelöste Verteilung des quantitativen Materialkoeffizienten berechnet werden. Die CT-Messdaten werden beispielsweise zumindest einem Tag vor dem Laden aus der Datenbank zur Berechnung der ortsaufgelösten Verteilung des quantitativen Materialkoeffizienten aus den CT-Messdaten akquiriert.

Eine Ausführungsform sieht vor, dass die Weiterverarbeitung der CT-Messdaten zusätzlich zum Berechnen der ortsaufgelösten Verteilung des quantitativen Materialkoeffizienten aus den spektral-aufgelösten CT-Messdaten eine Identifikation eines Zielvolumens und/oder Risikovolumens in den spektral-aufgelösten CT-Messdaten umfasst.

Demnach können die spektral-aufgelösten CT-Messdaten eine vorteilhafte Doppelfunktion erfüllen, nämlich sowohl als Grundlage für die Berechnung der ortsaufgelösten Verteilung des quantitativen Materialkoeffizienten als auch für die Identifikation des Zielvolumens und/oder Risikovolumens dienen. Die Akquisition der CT-Messdaten kann besonders geeignet auf die Identifikation des Zielvolumens und/oder Risikovolumens abgestimmt sein. Dies ist insbesondere dahingehend möglich, da die Akquisition der CT-Messdaten aufgrund der inhärenten spektralen Sensitivität des quantenzählenden Röntgendetektors keine besonderen Merkmale aufweisen muss, damit das Berechnen der ortsaufgelösten Verteilung des quantitativen Materialkoeffizienten aus den spektral-aufgelösten CT-Messdaten möglich ist.

Ein weiterer Vorteil der inhärenten spektralen Sensitivität der mittels des quantenzählenden Röntgendetektors akquirierten spektral-aufgelösten CT-Messdaten ist, dass Dual-Energy Applikationen standardmäßig in einer Weiterverarbeitung der spektral-aufgelösten CT-Messdaten eingesetzt werden können, ohne dass eine spezielle Dual-Energy Akquisition nötig ist. Derart kann auch erst nach dem Erfassen der spektral-aufgelösten CT-Messdaten festgelegt werden, dass eine Dual-Energy Applikation in der Weiterverarbeitung der spektral-aufgelösten CT-Messdaten eingesetzt werden soll. Dual-Energy Applikationen ermöglichen unterschiedliche Weiterverarbeitungen der spektral-aufgelösten CT-Messdaten und können beispielsweise zu einer verbesserten Differenzierung zwischen Knochengewebe und anderen Geweben führen. Weitere mögliche Dual-Energy Applikationen ermöglichen eine besonders vorteilhafte Auswertung in bestimmten Organregionen, wie beispielsweise im Knochenmarkt, in Blutgefäßen, im Gehirn, usw. Auch sind auf spezifische Krankheiten, wie beispielsweise der Gicht, abgestimmte Dual-Energy Applikationen denkbar.

Eine Ausführungsform sieht vor, dass die spektral-aufgelösten CT-Messdaten mittels derartiger Aufnahmeparameter des quantenzählenden Röntgendetektors akquiriert worden sind, dass die spektral-aufgelösten CT-Messdaten einen besonders geeigneten Kontrast zwischen verschiedenen Gewebetypen für die Identifikation des Zielvolumens und/oder Risikovolumens aufweisen.

Die spektral-aufgelösten CT-Messdaten werden demnach insbesondere derart akquiriert, dass sie besonders geeignet für die Identifikation des Zielvolumens und/oder Risikovolumens sind. Bei der Akquisition der spektral-aufgelösten CT-Messdaten steht demnach insbesondere die folgende Identifikation des Zielvolumens und/oder Risikovolumens im Vordergrund und nicht die Berechnung der ortsaufgelösten Verteilung des quantitativen Materialkoeffizienten, welche aufgrund der Verwendung inhärenten spektralen Sensitivität des quantenzählenden Röntgendetektors bei der Akquisition der CT-Messdaten typischerweise ohnehin möglich ist.

Hierbei ist das Vorgehen vorteilhaft, dass aus den spektral-aufgelösten CT-Messdaten die ortsaufgelöste Verteilung des linearen Abschwächungskoeffizienten für eine bestimmte Photonenenergie rekonstruiert wird. Die bestimmte Photonenenergie wird dabei vorteilhafterweise derart gewählt, dass die spektral-aufgelösten CT-Messdaten einen besonders geeigneten Kontrast zwischen den verschiedenen Gewebetypen für die Identifikation des Zielvolumens und/oder Risikovolumens aufweisen. Es ist auch denkbar, dass der lineare Abschwächungskoeffizient für verschiedene Energien rekonstruiert wird, um besonders geeignet zwischen verschiedenen Gewebetypen unterscheiden zu können. Hierbei ist auch eine Rekonstruktion denkbar, bei der die Energie, für welche der lineare Abschwächungskoeffizient bestimmt wird, ortsabhängig variiert. So kann lokal das bestmögliche Kontrast-zu-Rauschverhältnis für die Identifikation des Zielvolumens und/oder Risikovolumens erhalten werden.

Eine Ausführungsform sieht vor, dass die spektral-aufgelösten CT-Messdaten ein Messfeld abdecken, welches in einer axialen Messschicht sowohl den gesamten Körper des Patienten, als auch für die Lagerung des Patienten verwendete Positionierungshilfen umfasst.

Derart kann sichergestellt werden, dass die aus den spektral-aufgelösten CT-Messdaten berechnete ortsaufgelöste Verteilung des Materialkoeffizienten das genannte Messfeld abdeckt. Derart kann der quantitative Materialkoeffizient für das ausreichend große Messfeld (field of view, FOV) berechnet werden. Insbesondere ist es für die anschließende Dosisberechnung unter Verwendung der ortsaufgelösten Verteilung des Materialkoeffizienten wichtig, dass der Teil des Körpers des Patienten und diejenigen Positionierungshilfen sich im Messfeld, welche für die Bestrahlung des Patienten im Therapiestrahl positioniert sind.

Mittels der Verwendung des quantenzählenden Röntgendetektors bei der Datenakquisition können die spektral-aufgelösten CT-Messdaten besonders einfach für das gesamte Messfeld berechnet werden. Hierbei kann das gesamte Gesichtsfeld des CT-Geräts als Messfeld dienen. Im Gegensatz zu einer Dual-Energy Datenaufnahme gibt es keine weiteren Einschränkungen für das Messfeld, für welche die CT-Messdaten mit mehr als einer Energie akquiriert werden können. Weiterhin kann die Verwendung des quantenzählenden Röntgendetektors bei der Datenakquisition sicherstellen, dass die spektral-aufgelösten CT-Messdaten für das gesamte Messfeld mit konsistenter Genauigkeit akquiriert werden können. Derart kann eine besonders ackurate Berechnung des quantitativen Materialkoeffizienten für das gesamte Messfeld erzielt werden.

Eine Ausführungsform sieht vor, dass die Weiterverarbeitung der CT-Messdaten eine Strahlaufhärtungskorrektur der spektral-aufgelösten CT-Messdaten unter Verwendung von in den spektral-aufgelösten CT-Messdaten enthaltenen spektralen Informationen umfasst, wobei das Berechnen der ortsaufgelösten Verteilung des quantitativen Materialkoeffizienten aus den durch die Strahlaufhärtungskorrektur korrigierten spektral-aufgelösten CT-Messdaten erfolgt.

Mittels der in den spektral-aufgelösten CT-Messdaten enthaltenen spektralen Informationen kann die Strahlaufhärtung (engl. beam hardening) in den CT-Messdaten besonders vorteilhaft korrigiert werden. So kann die Strahlaufhärtungskorrektur der spektral-aufgelösten CT-Messdaten eine Dekomposition der spektral-aufgelösten CT-Messdaten in Wassergewebe und Knochengewebe unter Verwendung der in den spektral-aufgelösten CT-Messdaten enthaltenen spektralen Informationen umfassen. Diese Dekomposition kann übliche bei der Strahlaufhärtungskorrektur verwendete Schwellwert-Techniken vorteilhafterweise ersetzen oder ergänzen. Mittels der Strahlaufhärtungskorrektur können besonders vorteilhaft systematische Fehler bei der Berechnung des quantitativen Materialkoeffizienten vermieden werden. Derart kann vorteilhafterweise eine Genauigkeit einer Dosisberechnung mittels der derart berechneten ortsaufgelösten Verteilung des Materialkoeffizienten erhöht werden.

Eine Ausführungsform sieht vor, dass die Weiterverarbeitung der CT-Messdaten eine Identifikation eines Zielvolumens und/oder Risikovolumens in den CT-Messdaten umfasst, wobei das Bereitstellen der Ergebnisdaten ein Bereitstellen des identifizierten Zielvolumens und/oder Risikovolumens an die Schnittstelle umfasst.

Grundsätzlich kann unter der Identifikation des Zielvolumens und/oder Risikovolumens eine automatische, semi-automatische oder manuelle Segmentierung und/oder Konturierung des Zielvolumens und/oder Risikovolumens verstanden werden. Derart weisen die mittels dieser Weiterverarbeitung erzeugten Ergebnisdaten eine Information auf, welchen Raumbereich innerhalb der CT-Messdaten das identifizierte Zielvolumen und/oder Risikovolumen aufweist. Die Ergebnisdaten können auch eine Information aufweisen, welchen Verlauf eine äußere Begrenzung einer Kontur des identifizierten Zielvolumens und/oder Risikovolumens innerhalb der CT-Messdaten aufweist. Die Ergebnisdaten können demnach für die Planung der Bestrahlung nötige Informationen über die räumliche Lage des Zielvolumens und/oder Risikovolumens innerhalb der CT-Messdaten aufweisen. Selbstverständlich könne auch mehrere Zielvolumina und/oder mehrere Risikovolumina in den spektral-aufgelösten CT-Messdaten identifiziert werden.

Eine Ausführungsform sieht vor, dass die Identifikation des Zielvolumens und/oder Risikovolumens eine Abgrenzung eines Gewebes des Zielvolumens und/oder Risikovolumens von umliegendem Gewebe umfasst, wobei die Abgrenzung des Gewebes des Zielvolumens und/oder Risikovolumens vom umliegenden Gewebe unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

Die Verwendung des quantenzählenden Röntgendetektors bei der Akquisition der CT-Messdaten führt insbesondere dazu, dass jedes Röntgenphoton gleichermaßen zum Signal in den CT-Messdaten beiträgt. Derart ist das effektive in den mittels des quantenzählenden Röntgendetektors akquirierten CT-Messdaten detektierte Röntgenspektrum gegenüber mittels integrierender Detektoren akquirierten CT-Messdaten typischerweise zu niedrigeren Energien verschoben. Bei diesen niedrigeren Energien überwiegen vornehmlich Beitrage des Photoeffekts gegenüber Beiträgen des Compton-Effekts zur Absorption der Röntgenstrahlung. Die erhöhten Beiträge des Photoeffekts führen insbesondere dazu, dass höhere Unterschiede zwischen Materialien mit unterschiedlicher elementarer Zusammensetzung in den CT-Messdaten gemessen werden können. Dies wiederum führt vorteilhafterweise dazu, dass ein erhöhter Kontrast zwischen unterschiedlicher Gewebearten in den mittels des quantenzählenden Röntgendetektors akquirierten CT-Messdaten vorliegt. Gerade der Kontrast zwischen Gewebearten mit ähnlicher Elektronendichte, allerdings unterschiedlicher elementarer Zusammensetzung, insbesondere bezüglich Elementen mit höheren Ordnungszahlen, kann durch die Verwendung des quantenzählenden Röntgendetektors bei der Akquisition der CT-Messdaten erhöht sein. Derart kann mittels der derart akquirierten CT-Messdaten eine besonders deutliche Abgrenzung des Gewebes des Zielvolumens und/oder Risikovolumens vom umliegenden Gewebe erfolgen.

Eine Ausführungsform sieht vor, dass die Identifikation des Zielvolumens und/oder Risikovolumens eine Abgrenzung von Fettgewebe und Muskelgewebe umfasst, wobei die Abgrenzung des Fettgewebes und Muskelgewebes unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

Diesem Vorgehen liegt insbesondere die Überlegung zugrunde, dass die Verwendung des quantenzählenden Röntgendetektors bei der Akquisition der CT-Messdaten zu einem erhöhten Kontrast zwischen Fettgewebe und Muskelgewebe führt. Derart ist eine besonders geeignete Abgrenzung zwischen diesen zwei Gewebesorten in den CT-Messdaten möglich. Dagegen würden bei einer herkömmlichen Akquisition mittels eines integrierenden Röntgendetektors beide Gewebetypen ähnlich in den CT-Messdaten erscheinen, da sie eine ähnliche Elektronendichte aufweisen.

Eine Ausführungsform sieht vor, dass die Identifikation des Zielvolumens und/oder Risikovolumens eine Abgrenzung von grauer Gehirnsubstanz und weißer Gehirnsubstanz umfasst, wobei die Abgrenzung der grauen Gehirnsubstanz und weißen Gehirnsubstanz unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

Diesem Vorgehen liegt insbesondere die Überlegung zugrunde, dass die Verwendung des quantenzählenden Röntgendetektors bei der Akquisition der CT-Messdaten zu einem erhöhten Kontrast zwischen grauer Gehirnsubstanz und weißer Gehirnsubstanz führt. Derart ist eine besonders geeignete Abgrenzung zwischen diesen zwei Gewebesorten in den CT-Messdaten möglich. Dagegen würden bei einer herkömmlichen Akquisition mittels eines integrierenden Röntgendetektors beide Gewebetypen ähnlich in den CT-Messdaten erscheinen, da sie eine ähnliche Elektronendichte aufweisen.

Eine Ausführungsform sieht vor, dass das Erfassen der CT-Messdaten ein Erfassen von spektral-aufgelösten CT-Messdaten, welche sich aus der Akquisition mittels des quantenzählenden Röntgendetektors ergeben, umfasst, die Weiterverarbeitung der CT-Messdaten eine Strahlaufhärtungskorrektur der spektral-aufgelösten CT-Messdaten unter Verwendung von in den spektral-aufgelösten CT-Messdaten enthaltenen spektralen Informationen umfasst und die Identifikation des Zielvolumens und/oder Risikovolumens eine Abgrenzung von Knochenstrukturen in den durch die Strahlaufhärtungskorrektur korrigierten spektral-aufgelösten CT-Messdaten umfasst.

Wie bereits beschrieben kann die Strahlaufhärtungskorrektur besonders vorteilhaft in den spektral-aufgelösten CT-Messdaten durchgeführt werden. In den durch die Strahlaufhärtungskorrektur korrigierten spektral-aufgelösten CT-Messdaten können die Knochenstrukturen besonders gut gegeneinander abgegrenzt werden. Umfasst das Zielvolumen und/oder Risikovolumen eine Knochenstruktur bzw. einen unmittelbar benachbart zu einer Knochenstruktur gelegenen Körperbereich, ist derart eine verbesserte Identifikation des Zielvolumens und/oder Risikovolumens möglich.

Eine Ausführungsform sieht vor, dass die Knochenstrukturen in einer Schädelbasisregion des Patienten voneinander abgegrenzt werden.

Gerade die kleinen Knochenstrukturen in der Schädelbasisregion können in den durch die Strahlaufhärtungskorrektur korrigierten spektral-aufgelösten CT-Messdaten besonders gut voneinander abgegrenzt werden.

Eine Ausführungsform sieht vor, dass die CT-Messdaten einen ersten CT-Messdatensatz und einen zweiten CT-Messdatensatz umfassen, wobei der erste CT-Messdatensatz mittels einer höheren Energieschwelle des quantenzählenden Röntgendetektors als der zweite CT-Messdatensatz akquiriert worden ist, die Weiterverarbeitung der CT-Messdaten ein Erstellen einer gewichteten Kombination des ersten CT-Messdatensatzes und des zweiten CT-Messdatensatzes umfasst, wobei kombinierte CT-Messdaten erzeugt werden und die Identifikation des Zielvolumens und/oder Risikovolumens in den kombinierten CT-Messdaten erfolgt.

Das in dieser Ausführungsform beschriebene Vorgehen kann auch als Photonenwichtung bezeichnet werden. Der erste CT-Messdatensatz und der zweite CT-Messdatensatz werden insbesondere mittels unterschiedlicher Konfigurationen des quantenzählenden Röntgendetektors hinsichtlich der Energieschwellen akquiriert. Dass der erste CT-Messdatensatz mittels einer höheren Energieschwelle des quantenzählenden Röntgendetektors als der zweite CT-Messdatensatz akquiriert worden ist, kann insbesondere bedeuten, dass der erste CT-Messdatensatz im Mittel bei höheren Energieschwellen als der zweite CT-Messdatensatz akquiriert worden ist. Der erste CT-Messdatensatz weist demnach vorteilhafterweise Informationen über höher-energetische Photonen auf, als der zweite CT-Messdatensatz. Der erste CT-Messdatensatz und der zweite CT-Messdatensatz decken insbesondere das gleiche Messfeld ab.

Die mittels der Photonenwichtung erzeugten kombinierten CT-Messdaten sind insbesondere besonders gut für die Identifikation des Zielvolumens und/oder Risikovolumens geeignet. Die Informationen über unterschiedliche Photonenenergien aus dem ersten CT-Messdatensatz und dem zweiten CT-Messdatensatz können durch geeignete Wahl der Wichtungsfaktoren besonders vorteilhaft kombiniert werden, so dass eine verbesserte Identifikation des Zielvolumens und/oder Risikovolumens in den kombinierten CT-Messdaten möglich ist. Wichtungsfaktoren für die Bildung der gewichteten Kombination können in einer Nachverarbeitung basierend auf einem Inhalt des ersten CT-Messdatensatzes und/oder zweiten CT-Messdatensatzes generiert werden. Wie im Folgenden noch genauer beschrieben, können die Wichtungsfaktoren anhand einer groben Vorsegmentierung der CT-Messdaten bestimmt werden. Alternativ oder zusätzlich ist es auch denkbar, dass Wichtungsfaktoren, mittels welcher besonders für die Planung der Bestrahlung geeignete kombinierte CT-Messdaten erzeugt werden können, mittels eines maschinellen Lernverfahrens, beispielsweise eines künstlichen neuronalen Netzes, erhalten werden.

Eine Ausführungsform sieht vor, dass die Weiterverarbeitung der CT-Messdaten ein Extrahieren von Informationen für eine Strahlaufhärtungskorrektur aus dem ersten CT-Messdatensatz und eine Strahlaufhärtungskorrektur der kombinierten CT-Messdaten basierend auf den extrahierten Informationen umfasst, wobei die Identifikation des Zielvolumens und/oder Risikovolumens in den durch die Strahlaufhärtungskorrektur korrigierten kombinierten CT-Messdaten erfolgt.

Derart können die Informationen aus dem ersten CT-Messdatensatz, welcher insbesondere höher-energetische Photonen als der zweite CT-Messdatensatz charakterisiert, besonders geeignet für die Strahlaufhärtungskorrektur eingesetzt werden.

Eine Ausführungsform sieht vor, dass das Erstellen der gewichteten Kombination des ersten CT-Messdatensatzes und des zweiten CT-Messdatensatzes unter Einsatz von räumlich variierenden Wichtungsfaktoren erfolgt.

Derart wird insbesondere für die Erstellung der gewichteten Kombination an einem ersten Raumpunkt der CT-Messdaten ein anderer Wichtungsfaktor verwendet als für einen zweiten Raumpunkt der CT-Messdaten. Die Wichtungsfaktoren können beispielsweise über verschiedene Körperbereiche des Patienten variieren.

Eine Ausführungsform sieht vor, dass die Weiterverarbeitung der CT-Messdaten zunächst eine grobe Identifikation des Zielvolumens und/oder Risikovolumens umfasst, wobei die räumlich variierenden Wichtungsfaktoren anhand des grob segmentierten Zielvolumens und/oder Risikovolumens definiert werden und anschließend eine feine Identifikation des Zielvolumens und/oder Risikovolumens in den kombinierten CT-Messdaten erfolgt.

So kann beispielsweise für das Zielvolumen und/oder Risikovolumen ein unterschiedlicher Wichtungsfaktor gesetzt werden als für das Zielvolumen und/oder Risikovolumen umgebende Körperbereiche. Die grobe Identifikation des Zielvolumens und/oder Risikovolumens dient derart vorteilhafterweise der Bestimmung der Wichtungsfaktoren, mittels welchen dann eine besonders vorteilhafte Wichtung des ersten CT-Messdatensatzes und zweiten CT-Messdatensatzes erfolgen kann, so dass die feine Identifikation des Zielvolumens und/oder Risikovolumens in besonders geeignet gewichteten kombinierten CT-Messdaten erfolgen kann.

Eine Ausführungsform sieht vor, dass das Erfassen der CT-Messdaten eine Akquisition von hoch-aufgelösten CT-Messdaten mittels des quantenzählenden Röntgendetektors umfasst, wobei ein hoch-auflösender Modus des quantenzählenden Röntgendetektors zur Akquisition der hoch-aufgelösten CT-Messdaten eingesetzt wird, wobei in dem hoch-aufgelösten Modus jeder Pixel des quantenzählenden Röntgendetektors separat gezählt wird.

Typischerweise führt die Verwendung des quantenzählenden Röntgendetektors intrinsisch zu einer Erhöhung der Auflösung gegenüber konventionellen CT-Akquisitionen, da einzelne Detektorelemente, auch Detektorpixel genannt, des quantenzählenden Röntgendetektors kleiner als Detektorelemente von konventionellen CT-Detektoren sein müssen, da keine Trennschichten zwischen einzelnen Pixeln des quantenzählenden Röntgendetektors, welche eine Quanteneffizienz bei höherer Pixelauflösung verringern, nötig sind. Gleichzeitig liegt beim quantenzählenden Röntgendetektor üblicherweise nicht das Problem des Cross-Talks zwischen benachbarten Detektorpixeln vor, welcher typischerweise eine Verringerung der Größe der Detektorpixel von konventionellen CT-Detektoren erschwert.

Grundsätzlich kann somit mittels der Verwendung des quantenzählenden Röntgendetektors eine hohe Auflösung der CT-Messdaten in allen Raumrichtungen (x, y, z) und über das gesamte Messfeld, selbst bei der Verwendung eines großen Bore-Radius (large bore system), erzielt werden. Die Auflösung der akquirierten CT-Messdaten hängt dabei typischerweise von der Anzahl der Pixel des quantenzählenden Röntgendetektors, für welche die Zählimpulse gemeinsam gezählt werden, ab. In einem standardaufgelösten Modus des quantenzählenden Röntgendetektors werden dabei typischerweise mehrere Pixel des quantenzählenden Röntgendetektors gemeinsam gezählt, beispielsweise eine Pixelmatrix aus 2x2 Pixeln oder eine andersartige Zusammenschaltung von Pixeln. Die vorgeschlagene Verwendung des hoch-aufgelösten Modus (ultra-high resolution mode, UHR mode), in dem jeder Pixel des quantenzählenden Röntgendetektors separat gezählt wird, kann zu einer besonders hohen Auflösung der CT-Messdaten führen. Diese besonders hoch-aufgelösten CT-Messdaten können dann besonders vorteilhaft bei der Planung der Bestrahlung des Patienten verwendet werden.

Eine Ausführungsform sieht vor, dass die Weiterverarbeitung der CT-Messdaten ein Berechnen einer ortsaufgelösten Verteilung eines quantitativen Materialkoeffizienten aus den hoch-aufgelösten CT-Messdaten umfasst und das Bereitstellen der Ergebnisdaten ein Bereitstellen der ortsaufgelösten Verteilung des quantitativen Materialkoeffizienten an die Schnittstelle umfasst.

Die hoch-aufgelösten CT-Messdaten können besonders vorteilhaft in die Berechnung des quantitativen Materialkoeffizienten für Knochengewebe eingehen. Die hohe Auflösung der CT-Messdaten kann beispielsweise eine verbesserte Differenzierung zwischen unterschiedlichen Knochendichten des Knochengewebes ermöglichen.

Eine Ausführungsform sieht vor, dass die hoch-aufgelösten CT-Messdaten in das Berechnen der ortsaufgelösten Verteilung des quantitativen Materialkoeffizienten für ein Knochengewebe des Patienten in unveränderter Form eingehen und die hoch-aufgelösten CT-Messdaten in das Berechnen der ortsaufgelösten Verteilung des quantitativen Materialkoeffizienten für ein Weichteilgewebe des Patienten in einer reduzierten Auflösung eingehen.

Diesem Vorgehen liegt die Überlegung zugrunde, dass insbesondere für das Berechnen der ortsaufgelösten Verteilung des quantitativen Materialkoeffizienten für das Knochengewebe eine höhere Auflösung sinnvoll ist als für das Weichteilgewebe. Der Grund dafür ist, dass typischerweise im Knochengewebe feinere Strukturen zu unterscheiden sind als im Weichteilgewebe. Daher ist es häufig ausreichend, dass die hoch-aufgelösten CT-Messdaten für das Berechnen der ortsaufgelösten Verteilung des quantitativen Materialkoeffizienten für das Weichteilgewebe in ihrer Auflösung reduziert werden. Gemäß einer Ausführungsform wird die reduzierte Auflösung der hoch-aufgelösten CT-Messdaten mittels Filterung der hoch-aufgelösten CT-Messdaten unter Verwendung eines Filterkernels erzeugt.

Eine Ausführungsform sieht vor, dass das Berechnen der ortsaufgelösten Verteilung des quantitativen Materialkoeffizienten ein Berechnen einer ortsaufgelösten Verteilung eines Bremsvermögens aus den hoch-aufgelösten CT-Messdaten umfasst und das Bereitstellen der Ergebnisdaten ein Bereitstellen der ortsaufgelösten Verteilung des Bremsvermögens für eine Planung einer Partikel-Bestrahlung des Patienten umfasst.

Die Partikel-Bestrahlung erfolgt insbesondere mittels Protonen, Helium-Ionen oder Kohlenstoff-Ionen. Da für die Dosisberechnung bei einer Partikel-Bestrahlung typischerweise eine hohe Genauigkeit nötig ist, kann mittels der hoch-aufgelösten CT-Messdaten besonders vorteilhaft die ortsaufgelöste Verteilung des Bremsvermögens für die Planung der Partikel-Bestrahlung berechnet werden.

Eine Ausführungsform sieht vor, dass das Berechnen der ortsaufgelösten Verteilung des quantitativen Materialkoeffizienten aus den hoch-aufgelösten CT-Messdaten für zumindest eine der folgenden Körperregionen des Patienten erfolgt: eine Augenregion des Patienten, eine durch einen Verlauf des Trigeminusnervs des Patienten beschriebene Körperregion, eine Schädelbasis des Patienten, eine Lungenregion des Patienten.

Die hoch-aufgelösten CT-Messdaten können nämlich besonders vorteilhaft für die Planung der Bestrahlung von Körperregionen eingesetzt werden, für welche eine besonders hohe Genauigkeit nötig ist. Gerade die Verbindung mit dem Einsatz von einer besonders präzisen Bestrahlung der genannten Körperregionen, beispielsweise einer Partikel-Bestrahlung oder einer Brachytherapie-Bestrahlung, ist hierbei vorteilhaft möglich. Mittels der Verwendung der hoch-aufgelösten CT-Messdaten kann nämlich beispielsweise der Einfluss von kleinen Knochenstrukturen vorteilhafterweise bei der besonders präzisen Bestrahlung berücksichtigt werden.

Eine Ausführungsform sieht vor, dass das Erfassen der CT-Messdaten ein Erfassen von spektral-aufgelösten und zeitlich-aufgelösten CT-Messdaten, welche sich aus der Akquisition mittels des quantenzählenden Röntgendetektors ergeben, umfasst, die Weiterverarbeitung der CT-Messdaten ein Berechnen einer ortsaufgelösten und zeitlich-aufgelösten Verteilung eines quantitativen Materialkoeffizienten aus den spektral-aufgelösten und zeitlich-aufgelösten CT-Messdaten umfasst und das Bereitstellen der Ergebnisdaten ein Bereitstellen der ortsaufgelösten und zeitlich-aufgelösten Verteilung des quantitativen Materialkoeffizienten an die Schnittstelle umfasst.

Zeitlich-aufgelöste CT-Messdaten sind häufig wichtig für die Planung der Bestrahlung des Patienten, um eine Bewegung des Patienten, insbesondere eine zyklische Atembewegung, bei der Planung berücksichtigen zu können. Dies kann besonders wichtig sein, wenn das Zielvolumen in der Nähe von Organen, beispielsweise der Leber, liegt, welche sich besonders stark mit der Atembewegung bewegen.

Durch die Verwendung des quantenzählenden Röntgendetektors bei der Akquisition der CT-Messdaten kann sichergestellt werden, dass spektral-aufgelöste CT-Messdaten auch dynamisch über den Zeitverlauf der Atembewegung des Patienten akquiriert werden können. Diese spektral-aufgelösten CT-Messdaten können dabei eine volle zeitliche und räumliche Kohärenz über den Zeitverlauf der Atembewegung des Patienten aufweisen. Derart können vorteilhafterweise die genannten Dual-Energy Applikation in der Weiterverarbeitung der spektral-aufgelösten und zeitlich-aufgelösten CT-Messdaten eingesetzt werden. Beispielsweise können über den Zeitverlauf der Atembewegung des Patienten geeignet bestimmte Gewebe des Patienten in den CT-Messdaten hervorgehoben werden oder eine zeitaufgelöste Dosimetrie, insbesondere für ein in der Lunge gelegenes Zielvolumen für eine Partikeltherapie, ist denkbar. Auch kann in den zeitlich-aufgelösten CT-Messdaten eine Entfernung einer Kontrastmittelverstärkung (contrast removal) oder eine Strahlaufhärtungskorrektur erfolgen.

Eine Ausführungsform sieht vor, dass die spektral-aufgelösten und zeitlich-aufgelösten CT-Messdaten aus zumindest einer der folgenden Körperregionen des Patienten erfasst werden: eine Leberregion des Patienten, eine Pankreasregion des Patienten, einer Thoraxregion des Patienten.

Gerade bei einer, insbesondere Kontrastmittel-verstärkten, Bildgebung dieser Körperregionen können die spektral-aufgelösten und zeitlich-aufgelösten CT-Messdaten eine besonders vorteilhafte Grundlage für die Planung der Bestrahlung bieten.

Eine Ausführungsform sieht vor, dass das Erfassen der CT-Messdaten des Patienten die Akquisition der CT-Messdaten mittels des CT-Geräts, welches den quantenzählenden Röntgendetektor aufweist, umfasst, wobei während der Akquisition der CT-Messdaten ein Detektorparameter des quantenzählenden Röntgendetektors verändert wird.

Dass während der Akquisition der CT-Messdaten ein Detektorparameter des quantenzählenden Röntgendetektors verändert wird, bedeutet insbesondere, dass bei einem ersten Zeitpunkt der Akquisition der CT-Messdaten der quantenzählende Röntgendetektor mit einer unterschiedlichen Einstellung des Detektorparameters betrieben wird als bei einem zweiten Zeitpunkt der Akquisition der CT-Messdaten. Der Detektorparameter kann für verschiedene Abschnitte der Akquisition der CT-Messdaten verändert werden. Es ist auch eine dynamische kontinuierliche Veränderung des Detektorparameters während der Akquisition der CT-Messdaten denkbar.

Durch die statische oder dynamische Veränderung des Detektorparameters während der Datenakquisition können CT-Messdaten akquiriert werden, welche besonders geeignet für den Einsatz in der Planung der Bestrahlung des Patienten sind. Mögliche Anwendungsfälle sind in den folgenden Absätzen genauer beschrieben. An dieser Stelle sollen drei besonders geeignete Anwendungen erwähnt werden:
- Die Auflösung des quantenzählenden Röntgendetektors kann spezifisch für die Akquisition der CT-Messdaten in einem Körperbereich erhöht werden, in welchem die Begrenzungen eines zu konturierenden Organs erwartet werden.
- Der Detektorparameter kann derart angepasst werden, dass die Sensitivität bzw. der Weichgewebekontrast in einem Körperbereich, in welchem eine Anwesenheit eines Kontrastmittels zu erwarten ist, erhöht ist.
- In einem Körperbereich, in welchem das Vorliegen eines Knochengewebes zu erwarten ist, kann der Detektorparameter derart angepasst werden, dass eine verbesserte Dekomposition der Knochenmaterialien und/oder eine Reduktion von Strahlaufhärtungsartefakten erzielt werden kann.

Der genannte Detektorparameter kann auch nach der Akquisition der CT-Messdaten bei der Rekonstruktion der CT-Messdaten angepasst werden. So ist eine räumliche Variation der Auflösung und/oder von Energieschwellen bei der Rekonstruktion der CT-Messdaten denkbar. Es ist auch denkbar, dass Streifen von Detektorelementen des quantenzählenden Röntgendetektors mit unterschiedlichen Detektorparametern eingestellt werden. Dies kann spezifische Rekonstruktionen für eine spiralförmige Akquisition ermöglichen. Es ist auch denkbar, dass basierend auf dem Akquisitionsprotokoll und/oder einer Positionierung des Zielvolumens bzw. Risikovolumens für bestimmte Bereiche des quantenzählenden Röntgendetektors ausschließlich fixe Detektorparameter verwendet werden. Diese Fixierung der Detektorparameter kann bezüglich der Rotation des Detektor-Strahler-Systems und/oder einer Veränderung der Akquisition entlang der z-Position variieren.

Eine Ausführungsform sieht vor, dass während der Akquisition der CT-Messdaten eine Energieschwelle des quantenzählenden Röntgendetektors verändert wird.

Insbesondere können eine Energieschwelle des quantenzählenden Röntgendetektors oder auch mehrere Energieschwellen des quantenzählenden Röntgendetektors während der Akquisition der CT-Messdaten verändert werden. Wenn mehrere Energieschwellen des quantenzählenden Röntgendetektors während der Akquisition der CT-Messdaten verändert werden, wird insbesondere bei einem ersten Zeitpunkt der Akquisition der CT-Messdaten der quantenzählende Röntgendetektor mit einem anderen Satz von Energieschwellen betrieben als bei einem zweiten Zeitpunkt der Akquisition der CT-Messdaten. Dabei können unterschiedliche Photonenenergien in keV als Energieschwellen gesetzt werden. Die unterschiedlichen Energieschwellen können beispielsweise als Photonenenergie, als Spannung oder als Strom eingestellt werden.

Mittels der Anpassung der Energieschwelle kann der quantenzählende Röntgendetektor derart eingestellt werden, dass die CT-Messdaten besonders geeignet für die Planung der Bestrahlung akquiriert werden, beispielsweise lokal einen besonders geeigneten Kontrast zwischen verschiedenen Gewebetypen für die Identifikation des Zielvolumens und/oder Risikovolumens aufweisen.

Eine Ausführungsform sieht vor, dass während der Akquisition der CT-Messdaten eine Auflösung des quantenzählenden Röntgendetektors bezüglich einer Kombination von Pixeln des quantenzählenden Röntgendetektors verändert wird.

Derart kann der quantenzählende Röntgendetektor bei einem ersten Zeitpunkt der Akquisition der CT-Messdaten mit einer höheren Auflösung betrieben werden als bei einem zweiten Zeitpunkt der Akquisition der CT-Messdaten. Beim ersten Zeitpunkt werden demnach insbesondere weniger Pixel des quantenzählenden Röntgendetektors gemeinsam gezählt als beim zweiten Zeitpunkt.

Es ist insbesondere die Anwendung sinnvoll, dass hochaufgelöste CT-Messdaten von einem Knochengewebe des Patienten akquiriert werden, während niedrig-aufgelöste CT-Messdaten von einem Weichteilgewebe des Patienten akquiriert werden. Weiterhin ist auch eine höhere Auflösung der CT-Messdaten im Bereich des Zielvolumens und/oder Risikovolumens als im Rest des Körpers des Patienten sinnvoll.

Es ist weiterhin denkbar, dass während der Akquisition der CT-Messdaten ein Photonenfluss verändert wird. Der quantenzählende Röntgendetektor kann insbesondere über eine Anpassung einer Bittiefe der Übertragung an den veränderten Photonenfluss angepasst werden. So kann ein zu erwartender Photonenfluss, insbesondere gemäß einer eingestellten Modulation der Röntgenröhre, als Eingangsparameter für die Anpassung der Bittiefe verwendet werden. Beispielsweise kann bei Kenntnis einer maximal zu erwartenden Zählrate am quantenzählenden Röntgendetektor die Bittiefe der Übertragung entsprechend gedrosselt werden. Wird beispielsweise ein Photonenflussmaximum erwartet, so kann die Anzahl der zu übertragenden Schwellwerte und/oder Detektorzeilen und/oder Informationen über einzelne Detektorpixel erhöht werden. Der Photonenfluss kann nur in zumindest einer der drei Raumrichtungen (x, y, z) verändert werden. Es ist beispielsweise die vorteilhafte Anwendung denkbar, dass in der Nähe des Zielvolumens der Bestrahlung die Auflösung des quantenzählenden Röntgendetektors erhöht wird und gleichzeitig eine besonders hohe Dosisleistung für die Akquisition aus diesem Bereich gesetzt wird. Derart kann genügend Dosis für die Größe der Pixel des quantenzählenden Röntgendetektors vorliegen.

Weiterhin ist auch denkbar, dass während der Akquisition der CT-Messdaten ein linearer Korrekturparameter des quantenzählenden Röntgendetektors verändert wird.

Es sind unterschiedliche Arten denkbar, auf welche Weise der Detektorparameter während der Akquisition der CT-Messdaten verändert wird. Das grundlegende Ziel ist insbesondere, dass für unterschiedliche Körperregionen jeweils CT-Messdaten mit einer möglich geeigneten Einstellung des Detektorparameters akquiriert werden.

Gemäß einer ersten Ausführungsform wird der Detektorparameter des quantenzählenden Röntgendetektors während der Akquisition der CT-Messdaten derart verändert, dass für eine Akquisition der CT-Messdaten an einer ersten z-Position entlang einer Längsrichtung des Patienten eine erste Einstellung des Detektorparameters gesetzt wird und für eine Akquisition der CT-Messdaten an einer zweiten z-Position entlang der Längsrichtung des Patienten eine zweite Einstellung des Detektorparameters gesetzt wird. Gemäß einer zweiten Ausführungsform wird der Detektorparameter des quantenzählenden Röntgendetektors während der Akquisition der CT-Messdaten derart verändert, dass für eine Akquisition der CT-Messdaten bei einer ersten Rotationsposition eines Detektor-Strahler-Systems des CT-Geräts eine erste Einstellung des Detektorparameters gesetzt wird und für eine Akquisition der CT-Messdaten bei einer zweiten Rotationsposition des Detektor-Strahler-Systems des CT-Geräts eine zweite Einstellung des Detektorparameters gesetzt wird. Gemäß einer dritten Ausführungsform wird der Detektorparameter des quantenzählenden Röntgendetektors während der Akquisition der CT-Messdaten derart verändert, dass für eine Akquisition der CT-Messdaten einer ersten Körperregion des Patienten eine erste Einstellung des Detektorparameters gesetzt wird und für eine Akquisition der CT-Messdaten einer zweiten Körperregion des Patienten eine zweite Einstellung des Detektorparameters gesetzt wird.

In den folgenden Ausführungsformen wird die Möglichkeit näher beschrieben, dass für die Akquisition der CT-Messdaten der ersten Körperregion des Patienten die erste Einstellung des Detektorparameters gesetzt wird und für die Akquisition der CT-Messdaten der zweiten Körperregion des Patienten die zweite Einstellung des Detektorparameters gesetzt wird. Die Ausführungen lassen sich weitgehend auch auf die anderen Möglichkeiten übertragen.

Eine Ausführungsform sieht vor, dass die erste Körperregion und die zweite Körperregion vor der Akquisition der CT-Messdaten anhand von bereits vom Patienten vorliegenden medizinischen Bilddaten und/oder unter Verwendung einer Atlas-Datenbank festgelegt werden.

Für die bereits vom Patienten vorliegenden medizinischen Bilddaten kann bereits eine Kontur einer Organstruktur vorliegen. Die Kontur der Organstruktur kann bei dem Festlegen der ersten Körperregion und der zweiten Körperregion verwendet werden. Es ist auch denkbar, dass die Kontur der Organstruktur basierend auf in der Atlas-Datenbank enthaltenen Informationen festgelegt wird. Durch diese Vorgehensweise kann für die Akquisition der CT-Messdaten aus der Organstruktur ein anderer Detektorparameter gesetzt werden als für die Akquisition der übrigen CT-Messdaten. Gemäß einer Ausführungsform umfasst die erste Körperregion ein Zielvolumen der Bestrahlung und die zweite Körperregion umfasst eine außerhalb des Zielvolumens der Bestrahlung liegende Körperregion.

Eine Ausführungsform sieht vor, dass die erste Körperregion derart ausgebildet ist, dass sie bei der Bestrahlung des Patienten in einem Strahlbereich positioniert ist, und die zweite Körperregion derart ausgebildet ist, dass sie bei der Bestrahlung des Patienten außerhalb eines Strahlbereichs positioniert ist.

Der Strahlbereich ist insbesondere aus Strahleinstellungen des Strahlentherapiegeräts, welche durch einen bereits existierenden Bestrahlungsplan festgelegt werden, bekannt.

Eine Ausführungsform sieht vor, dass die erste Körperregion und die zweite Körperregion derart ausgebildet sind, dass bei der Bestrahlung des Patienten für die erste Körperregion eine höhere Strahlendosis vorgesehen ist als für die zweite Körperregion.

Eine bekannte dreidimensionale Dosisverteilung aus einem bereits bestehenden Bestrahlungsplan kann derart als Eingangsinformation für das Festlegen des Detektorparameters verwendet werden. Der Detektorparameter kann dann derart festgelegt werden, dass besonders für eine Optimierung bzw. Anpassung des bereits bestehenden Bestrahlungsplans geeignete CT-Messdaten akquiriert werden können. Diesem Vorgehen liegt insbesondere die Überlegung zugrunde, dass für die erste Körperregion, für welche eine höhere Strahlendosis vorgesehen ist, eine genauere Bildgebung nötig ist als für die zweite Körperregion, für welche eine geringere Strahlendosis vorgesehen ist. Auch ist es denkbar, dass die CT-Messdaten eine höhere Präzision für diejenige Körperregion aufweisen müssen, in welcher ein steiler Dosisgradient vorliegt, beispielsweise nahe dem Bragg-Peak bei einer Partikeltherapie oder nahe einer Position von Brachytherapie-Applikatoren oder in der Nähe des Zielvolumens bzw. Risikovolumens.

Eine Ausführungsform sieht vor, dass während der Akquisition der CT-Messdaten der Detektorparameter des quantenzählenden Röntgendetektors derart verändert wird, dass für die Akquisition der CT-Messdaten aus der ersten Körperregion des Patienten eine höhere Auflösung des quantenzählenden Röntgendetektors gesetzt wird als für die Akquisition der CT-Messdaten aus der zweiten Körperregion des Patienten.

Diesem vorteilhaften Vorgehen liegt die Überlegung zugrunde, dass für die erste Körperregion höher aufgelöste CT-Messdaten sinnvoll sind als für die zweite Körperregion.

Alternativ oder zusätzlich ist es auch denkbar, dass externe Kameradaten, beispielsweise 3D-Kameradaten, verwendet werden, um die Körperregion, beispielsweise die Nacken-Schulter-Region, festzulegen, in welcher der Detektorparameter für die Akquisition mittels des quantenzählenden Röntgendetektors angepasst werden soll.

Eine Ausführungsform sieht vor, dass die Schnittstelle weitere Messdaten erfasst, welche mittels einer Bildgebungsvorrichtung eines für die Bestrahlung des Patienten eingesetzten Strahlentherapiegeräts erfasst werden, wobei für die Planung der Bestrahlung des Patienten die Ergebnisdaten in Bezug zu den weiteren Messdaten gesetzt werden.

Durch die Verwendung des quantenzählenden Röntgendetektors bei der Akquisition der CT-Messdaten können die Ergebnisdaten leichter in den Bezug zu den weiteren Messdaten gesetzt werden. Beispielsweise ist eine einfachere Streustrahlungskorrektur möglich.

Eine Ausführungsform sieht vor, dass die Bildgebungsvorrichtung des für die Bestrahlung des Patienten eingesetzten Strahlentherapiegeräts ebenfalls einen quantenzählenden Röntgendetektor aufweist, wobei die Ergebnisdaten unter Verwendung eines spezifische Informationsgehalts der CT-Messdaten und der weiteren CT-Messdaten, welcher sich aus der Verwendung des quantenzählenden Röntgendetektors bei der Akquisition der CT-Messdaten und der weiteren Messdaten ergibt, in Bezug zu den weiteren Messdaten gesetzt werden.

Unter Verwendung des zusätzlichen spezifischen Informationsgehalts können die CT-Messdaten besonders einfach in Bezug zu den weiteren Messdaten gesetzt werden.

Eine Ausführungsform sieht vor, dass die CT-Messdaten zumindest zwei CT-Messdatensätze umfasst, welche zu unterschiedlichen Zeitpunkten erfasst worden sind, wobei die zumindest zwei CT-Messdatensätze unter Verwendung des spezifischen Informationsgehalts der CT-Messdaten, welcher sich aus der Verwendung des zumindest einen quantenzählenden Röntgendetektors bei der Akquisition der CT-Messdaten ergibt, registriert werden, wobei die registrierten zumindest zwei CT-Messdatensätze an die Schnittstelle bereitgestellt werden.

Derart kann eine Registrierung der zumindest zwei CT-Messdatensätze verbessert werden. Dies kann für spezielle CT-Applikationen, beispielsweise eine Perfusionsbildgebung bzw. eine funktionelle Bildgebung, insbesondere der Lunge des Patienten, sinnvoll sein.

In den folgenden zwei Ausführungsformen sind noch zwei Anwendungsfälle beschrieben, bei denen eine Kontrastmittelbasierte Akquisition der CT-Messdaten mittels des quantenzählenden Röntgendetektors geeignet durchgeführt und ausgewertet werden kann: Die erste Ausführungsform sieht vor, dass während der Akquisition der CT-Messdaten ein Kontrastmittel eingesetzt worden ist, wobei die Weiterverarbeitung der CT-Messdaten eine Materialdekomposition der CT-Messdaten in Gewebe, welches Kontrastmittel aufweist, und übriges Gewebe unter Verwendung des spezifischen Informationsgehalts der CT-Messdaten, welcher sich aus der Verwendung des zumindest einen quantenzählenden Röntgendetektors bei der Akquisition der CT-Messdaten ergibt, umfasst. Die zweite Ausführungsform sieht vor, dass während der Akquisition der CT-Messdaten ein Kontrastmittel eingesetzt worden ist, wobei die Weiterverarbeitung der CT-Messdaten eine Erstellung eines virtuellen nicht-kontrastierten CT-Bildes unter Verwendung des spezifischen Informationsgehalts der CT-Messdaten, welcher sich aus der Verwendung des zumindest einen quantenzählenden Röntgendetektors bei der Akquisition der CT-Messdaten ergibt, umfasst. Hierbei kann eine k-edge Bildgebung eingesetzt werden, um das virtuelle nicht-kontrastierte Bild zu erstellen.

Schließlich ist es auch denkbar, dass die Weiterverarbeitung der CT-Messdaten eine Erkennung und/oder Quantifizierung von Metall-Nanopartikeln, welche sich im Patienten zur Erhöhung eines Effekts der Bestrahlung des Patienten befinden, unter Verwendung des spezifischen Informationsgehalts der CT-Messdaten, welcher sich aus der Verwendung des zumindest einen quantenzählenden Röntgendetektors bei der Akquisition der CT-Messdaten ergibt, umfasst.

Die erfindungsgemäße Recheneinheit umfasst zumindest ein Rechenmodul, wobei die Recheneinheit zum Ausführen eines erfindungsgemäßen Verfahrens ausgebildet ist.

Derart ist die Recheneinheit ausgebildet zum Ausführen eines Verfahrens zum Bereitstellen von Ergebnisdaten, welche geeignet für einen Einsatz in einer Planung einer Bestrahlung eines Patienten sind. Das Rechenmodul ist zum Erfassen von CT-Messdaten des Patienten, welche mittels eines CT-Geräts, welches einen quantenzählenden Röntgendetektor aufweist, akquiriert worden sind, zur Weiterverarbeitung der CT-Messdaten, wobei bei der Weiterverarbeitung der CT-Messdaten ein spezifischer Informationsgehalt der CT-Messdaten, welcher sich aus der Verwendung des quantenzählenden Röntgendetektors bei der Akquisition der CT-Messdaten ergibt, berücksichtigt wird, wobei bei der Weiterverarbeitung der CT-Messdaten Ergebnisdaten erzeugt werden, welche geeignet für den Einsatz in der Planung der Bestrahlung des Patienten sind, und zum Bereitstellen der Ergebnisdaten an eine Schnittstelle, so dass die Ergebnisdaten für die Planung der Bestrahlung des Patienten verwendet werden können, ausgebildet.

Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Das erfindungsgemäße CT-Gerät umfasst die erfindungsgemäße Recheneinheit.

Die Recheneinheit kann dazu ausgebildet sein, Steuerungssignale an das CT-Gerät zu senden und/oder Steuerungssignale zu empfangen und/oder zu verarbeiten, um ein erfindungsgemäßes Verfahren auszuführen. Die Recheneinheit kann in das CT-Gerät integriert sein. Die Recheneinheit kann auch separat von dem CT-Gerät installiert sein. Die Recheneinheit kann mit dem CT-Gerät verbunden sein.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Die Vorteile der erfindungsgemäßen Recheneinheit, des erfindungsgemäßen CT-Geräts und des erfindungsgemäßen Computerprogrammprodukts entsprechen im Wesentlichen den Vorteilen des erfindungsgemäßen Verfahrens, welche vorab im Detail ausgeführt sind. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert.

Es zeigen:
- Fig. 1: ein erfindungsgemäßes CT-Gerät mit einer erfindungsgemäßen Recheneinheit,
- Fig. 2: ein Ablaufdiagramm einer ersten Ausführungsform eines erfindungsgemäßen Verfahrens,
- Fig. 3: ein Ablaufdiagramm einer zweiten Ausführungsform eines erfindungsgemäßen Verfahrens,
- Fig. 4: ein Ablaufdiagramm einer dritten Ausführungsform eines erfindungsgemäßen Verfahrens,
- Fig. 5: ein Ablaufdiagramm einer vierten Ausführungsform eines erfindungsgemäßen Verfahrens,
- Fig. 6: ein Ablaufdiagramm einer fünften Ausführungsform eines erfindungsgemäßen Verfahrens,
- Fig. 7: ein Ablaufdiagramm einer sechsten Ausführungsform eines erfindungsgemäßen Verfahrens,
- Fig. 8: ein Ablaufdiagramm einer siebten Ausführungsform eines erfindungsgemäßen Verfahrens,
- Fig. 9: ein Ablaufdiagramm einer achten Ausführungsform eines erfindungsgemäßen Verfahrens,
- Fig. 10: ein Ablaufdiagramm einer neunten Ausführungsform eines erfindungsgemäßen Verfahrens und
- Fig. 11: ein Ablaufdiagramm einer zehnten Ausführungsform eines erfindungsgemäßen Verfahrens.

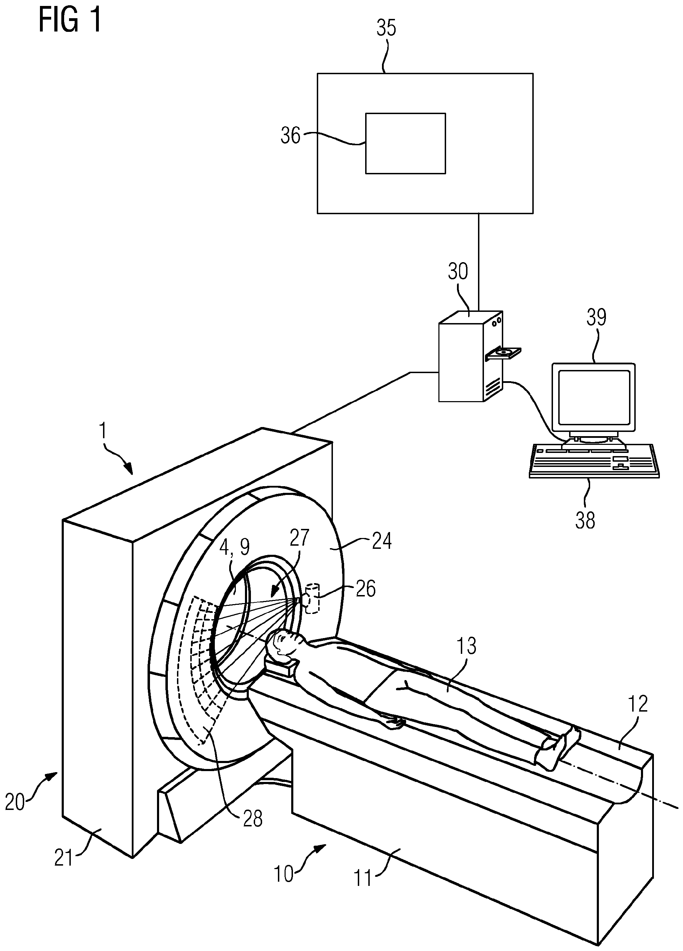

**Fig. 1** zeigt ein erfindungsgemäßes CT-Gerät 1 mit einer erfindungsgemäßen Recheneinheit 35.

Das CT-Gerät 1 weist eine Gantry 20, eine tunnelförmige Öffnung 9, eine Patientenlagerungsvorrichtung 10 und eine Steuerungsvorrichtung 30 auf. Die Gantry 20 weist einen stationären Tragrahmen 21 und einen Rotor 24 auf. Der Rotor 24 ist mittels einer Drehlagerungsvorrichtung um eine Rotationsachse relativ zu dem stationären Tragrahmen 21 drehbar an dem stationären Tragrahmen 21 angeordnet. In die tunnelförmige Öffnung 9 ist ein Patient 13 einführbar. In der tunnelförmigen Öffnung 9 befindet sich ein Akquisitionsbereich 4. In dem Akquisitionsbereich 4 ist ein abzubildender Bereich des Patienten 13, insbesondere das Messfeld, derart positionierbar, dass eine elektromagnetische Strahlung 27 von einer Strahlungsquelle 26 zu dem abzubildenden Bereich und nach einer Wechselwirkung mit dem abzubildenden Bereich zu einem quantenzählenden Röntgendetektor 28 gelangen kann. Die Patientenlagerungsvorrichtung 10 weist einen Lagerungstisch 11 und eine Transferplatte 12 zur Lagerung des Patienten 13 auf. Die Transferplatte 12 ist derart relativ zu dem Lagerungstisch 11 bewegbar an dem Lagerungstisch 11 angeordnet, dass die Transferplatte 12 in einer Längsrichtung der Transferplatte 12 in den Akquisitionsbereich 4 einführbar ist.

Das CT-Gerät 1 ist zur Akquisition von CT-Messdaten basierend auf der elektromagnetischen Strahlung 27 ausgebildet. Das CT-Gerät 1 umfasst eine CT-Messdaten-Akquisitionseinheit mit der Strahlungsquelle 26, insbesondere einer Röntgenquelle, und dem quantenzählenden Röntgendetektor 28. Die Strahlungsquelle 26 ist an dem Rotor 24 angeordnet und zur Emission einer Strahlung 27, insbesondere einer Röntgenstrahlung, mit Strahlungsquanten 27 ausgebildet. Der quantenzählende Röntgendetektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den quantenzählenden Röntgendetektor 28 auftreffen. Auf diese Weise können mittels der Erfassungseinheit CT-Messdaten des abzubildenden Bereichs erfasst werden.

Eine Steuerungsvorrichtung 30 ist zum Empfangen und Weiterverarbeiten der von der Erfassungseinheit erfassten CT-Messdaten ausgebildet. Die Steuerungsvorrichtung 30 ist zum Steuern des CT-Geräts 1 ausgebildet.

Das CT-Gerät 1 weist eine Eingabeeinheit 38 und eine Anzeigeeinheit 39 auf, welche jeweils mit der Steuerungsvorrichtung 30 verbunden sind. Die Eingabeeinheit 38 ist zum Eingeben von Steuerungs-Informationen, z. B. Bildrekonstruktionsparametern und/oder Untersuchungsparametern, ausgebildet. Die Anzeigeeinheit 39 ist insbesondere zum Anzeigen der akquirierten CT-Messdaten, insbesondere von aus den CT-Messdaten rekonstruierten CT-Bilddaten ausgebildet.

Das dargestellte CT-Gerät 1 kann selbstverständlich weitere Komponenten umfassen, die CT-Geräte 1 gewöhnlich aufweisen. Eine allgemeine Funktionsweise eines CT-Geräts 1 ist zudem dem Fachmann bekannt, so dass auf eine detaillierte Beschreibung der weiteren Komponenten verzichtet wird.

Die dargestellte Recheneinheit 35 umfasst zumindest ein Rechenmodul 36. Das CT-Gerät 1 ist somit zusammen mit der Recheneinheit 35 zur Ausführung eines erfindungsgemäßen Verfahrens ausgelegt. Die Recheneinheit kann die CT-Messdaten von der Steuerungsvorrichtung 30 des CT-Gerät 1 erfassen. Dafür ist die Recheneinheit 35 vorteilhafterweise mit der Steuerungsvorrichtung 30 des CT-Geräts 11 hinsichtlich eines Datenaustauschs verbunden. Alternativ kann die Recheneinheit 35 auch alleine zur Ausführung eines erfindungsgemäßen Verfahrens ausgelegt sein. Dazu wird die Recheneinheit 35 typischerweise die CT-Messdaten aus einer Datenbank laden und/oder von dem CT-Gerät 1 abrufen.

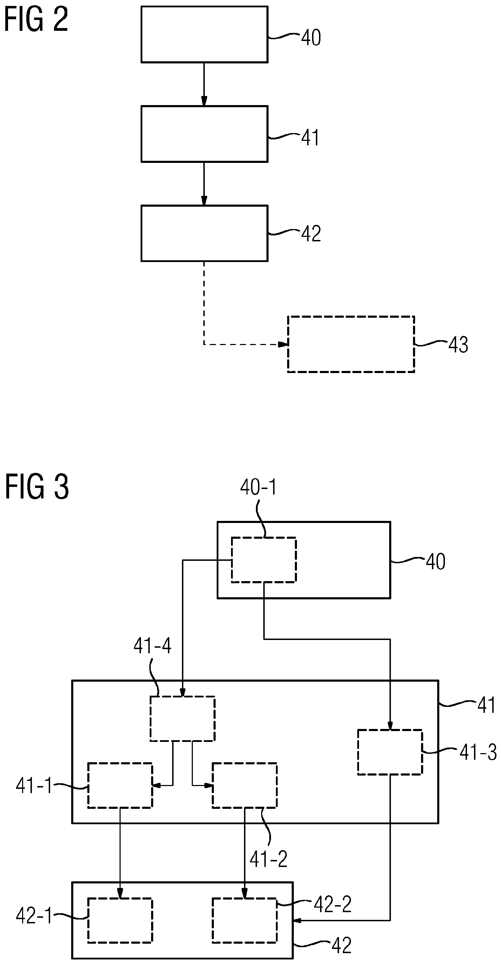

**Fig. 2** zeigt ein Ablaufdiagramm einer ersten Ausführungsform eines erfindungsgemäßen Verfahrens zum Bereitstellen von Ergebnisdaten, welche geeignet für einen Einsatz in einer Planung einer Bestrahlung eines Patienten sind.

In einem Verfahrensschritt 40 erfolgt ein Erfassen von CT-Messdaten des Patienten, welche mittels eines CT-Geräts, welches einen quantenzählenden Röntgendetektor aufweist, akquiriert worden sind.

In einem Verfahrensschritt 41 erfolgt eine Weiterverarbeitung der CT-Messdaten, wobei bei der Weiterverarbeitung der CT-Messdaten ein spezifischer Informationsgehalt der CT-Messdaten, welcher sich aus der Verwendung des quantenzählenden Röntgendetektors bei der Akquisition der CT-Messdaten ergibt, berücksichtigt wird, wobei bei der Weiterverarbeitung der CT-Messdaten Ergebnisdaten erzeugt werden, welche geeignet für den Einsatz in der Planung der Bestrahlung des Patienten sind.

In einem Verfahrensschritt 42 erfolgt ein Bereitstellen der Ergebnisdaten an eine Schnittstelle, so dass die Ergebnisdaten für die Planung der Bestrahlung des Patienten verwendet werden können.

Das erfindungsgemäße Verfahren kann derart erweitert werden, dass in einem Verfahrensschritt 43 eine Durchführen der Planung der Bestrahlung des Patienten erfolgt, wobei die Ergebnisdaten von der Schnittstelle abgerufen werden und bei der Planung der Bestrahlung des Patienten eingesetzt werden.

Die nachfolgende Beschreibung beschränkt sich im Wesentlichen auf die Unterschiede zu dem Ausführungsbeispiel in Fig. 2, wobei bezüglich gleich bleibender Verfahrensschritte auf die Beschreibung des Ausführungsbeispiels in Fig. 2 verwiesen wird. Im Wesentlichen gleich bleibende Verfahrensschritte sind grundsätzlich mit den gleichen Bezugszeichen beziffert.

**Fig. 3** zeigt ein Ablaufdiagramm einer zweiten Ausführungsform eines erfindungsgemäßen Verfahrens.

Das Erfassen der CT-Messdaten im Verfahrensschritt 40 umfasst in einem Verfahrensschritt 40-1 ein Erfassen von spektral-aufgelösten CT-Messdaten, welche sich aus der Akquisition mittels des quantenzählenden Röntgendetektors ergeben. Alternativ oder zusätzlich kann das Erfassen der CT-Messdaten ein Laden von zuvor akquirierten spektral-aufgelösten CT-Messdaten aus einer Datenbank zur Berechnung der ortsaufgelösten Verteilung des quantitativen Materialkoeffizienten umfassen. Die spektral-aufgelösten CT-Messdaten decken vorteilhafterweise ein Messfeld ab, welches in einer axialen Messschicht sowohl den gesamten Körper des Patienten, als auch für die Lagerung des Patienten verwendete Positionierungshilfen umfasst.

Die Weiterverarbeitung der CT-Messdaten im Verfahrensschritt 41 umfasst ein Berechnen einer ortsaufgelösten Verteilung eines quantitativen Materialkoeffizienten aus den spektral-aufgelösten CT-Messdaten in einem Verfahrensschritt 41-1. Das Bereitstellen der Ergebnisdaten umfasst ein Bereitstellen der ortsaufgelösten Verteilung des quantitativen Materialkoeffizienten an die Schnittstelle in einem Verfahrensschritt 42-1.

In Fig. 3 ist dabei das vorteilhafte Vorgehen dargestellt, dass die Weiterverarbeitung der CT-Messdaten eine Berechnung von zwei ortsaufgelösten Verteilungen von zwei unterschiedlichen quantitativen Materialkoeffizienten umfasst. So wird im Verfahrensschritt 41-1 eine erste ortsaufgelösten Verteilung eines ersten quantitativen Materialkoeffizienten und in einem Verfahrensschritt 41-2 eine zweite ortsaufgelösten Verteilung eines zweiten quantitativen Materialkoeffizienten aus den spektral-aufgelösten CT-Messdaten berechnet. Dementsprechend umfasst das Bereitstellen der Ergebnisdaten im Verfahrensschritt 42 gemäß Fig. 3 ein Bereitstellen der ersten ortsaufgelösten Verteilung des ersten quantitativen Materialkoeffizienten für einen ersten Dosisberechnungsalgorithmus in einem Verfahrensschritt 42-1 und ein Bereitstellen der zweiten ortsaufgelösten Verteilung des zweiten quantitativen Materialkoeffizienten für einen zweiten Dosisberechnungsalgorithmus in einem Verfahrensschritt 42-2. Beispielsweise ist dabei der erste quantitative Materialkoeffizient eine Elektronendichte und der zweite quantitative Materialkoeffizient ist eine effektive Kernladungszahl. Es ist auch denkbar, dass der erste quantitative Materialkoeffizient und der zweite quantitative Materialkoeffizient auf zwei voneinander linear-unabhängigen Basismaterialien basieren.

Nicht in Fig. 3 gezeigt ist das vorteilhafte Vorgehen, dass die Weiterverarbeitung der CT-Messdaten eine getrennte Weiterverarbeitung der ersten ortsaufgelösten Verteilung des ersten quantitativen Materialkoeffizienten und der zweiten ortsaufgelösten Verteilung des zweiten quantitativen Materialkoeffizienten und eine Erzeugung der Ergebnisdaten mittels einer Zusammenführung der bei der getrennten Weiterverarbeitung erzeugten Teil-Ergebnisdaten umfasst.

In Fig. 3 ist ebenfalls das Vorgehen dargestellt, dass die Weiterverarbeitung der CT-Messdaten in einem Verfahrensschritt 41-3 zusätzlich zum Berechnen der ortsaufgelösten Verteilung des quantitativen Materialkoeffizienten aus den spektral-aufgelösten CT-Messdaten eine Identifikation eines Zielvolumens und/oder Risikovolumens in den spektral-aufgelösten CT-Messdaten umfasst. Hierbei ist das vorteilhafte Vorgehen denkbar, dass die spektral-aufgelösten CT-Messdaten mittels derartiger Aufnahmeparameter des quantenzählenden Röntgendetektors akquiriert worden sind, dass die spektral-aufgelösten CT-Messdaten einen besonders geeigneten Kontrast zwischen verschiedenen Gewebetypen für die Identifikation des Zielvolumens und/oder Risikovolumens aufweisen.

Im in Fig. 3 gezeigten Fall umfasst die Weiterverarbeitung der CT-Messdaten in einem Verfahrensschritt 41-4 eine Strahlaufhärtungskorrektur der spektral-aufgelösten CT-Messdaten unter Verwendung von in den spektral-aufgelösten CT-Messdaten enthaltenen spektralen Informationen, wobei das Berechnen der ortsaufgelösten Verteilung des quantitativen Materialkoeffizienten aus den durch die Strahlaufhärtungskorrektur korrigierten spektral-aufgelösten CT-Messdaten erfolgt. Selbstverständlich kann entgegen dem in Fig. 3 gezeigten Vorgehen der quantitative Materialkoeffizient auch direkt aus den nicht korrigierten CT-Messdaten berechnet werden.

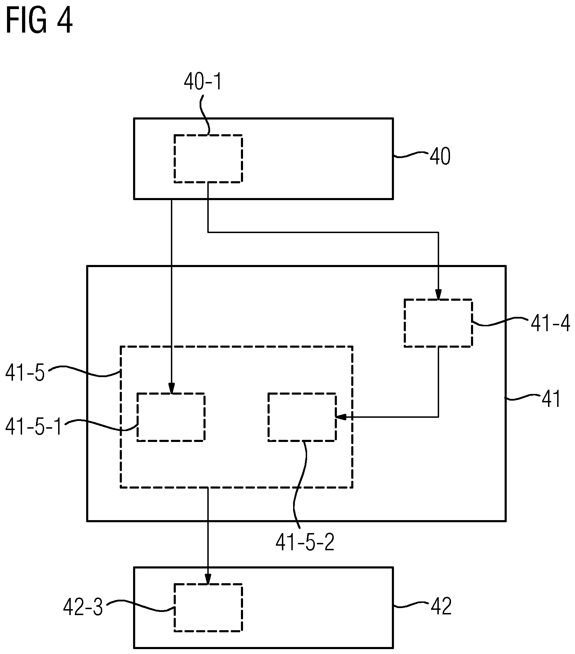

**Fig. 4** zeigt ein Ablaufdiagramm einer dritten Ausführungsform eines erfindungsgemäßen Verfahrens.

Im in Fig. 4 gezeigten Fall umfasst die Weiterverarbeitung der CT-Messdaten in einem Verfahrensschritt 41-5 eine Identifikation eines Zielvolumens und/oder Risikovolumens in den CT-Messdaten. Derart umfasst das Bereitstellen der Ergebnisdaten ein Bereitstellen des identifizierten Zielvolumens und/oder Risikovolumens an die Schnittstelle in einem Verfahrensschritt 42-3 umfasst.

Hierbei umfasst im Fig. 4 gezeigten Fall die Identifikation des Zielvolumens und/oder Risikovolumens in einem Verfahrensschritt 41-5-1 eine Abgrenzung eines Gewebes des Zielvolumens und/oder Risikovolumens von umliegendem Gewebe umfasst, wobei die Abgrenzung des Gewebes des Zielvolumens und/oder Risikovolumens vom umliegenden Gewebe unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

Es ist auch das vorteilhafte Vorgehen denkbar, dass die Identifikation des Zielvolumens und/oder Risikovolumens eine Abgrenzung von Fettgewebe und Muskelgewebe umfasst, wobei die Abgrenzung des Fettgewebes und Muskelgewebes unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

Alternativ oder zusätzlich ist auch das Vorgehen denkbar, dass die Identifikation des Zielvolumens und/oder Risikovolumens eine Abgrenzung von grauer Gehirnsubstanz und weißer Gehirnsubstanz umfasst, wobei die Abgrenzung der grauen Gehirnsubstanz und weißen Gehirnsubstanz unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

Zusätzlich ist in Fig. 4 das Vorgehen dargestellt, dass in einem Verfahrensschritt 40-1 das Erfassen der CT-Messdaten ein Erfassen von spektral-aufgelösten CT-Messdaten, welche sich aus der Akquisition mittels des quantenzählenden Röntgendetektors ergeben, umfasst, in einem Verfahrensschritt 41-4 die Weiterverarbeitung der CT-Messdaten eine Strahlaufhärtungskorrektur der spektral-aufgelösten CT-Messdaten unter Verwendung von in den spektral-aufgelösten CT-Messdaten enthaltenen spektralen Informationen umfasst und in einem Verfahrensschritt 41-5-2 die Identifikation des Zielvolumens und/oder Risikovolumens eine Abgrenzung von Knochenstrukturen in den durch die Strahlaufhärtungskorrektur korrigierten spektral-aufgelösten CT-Messdaten umfasst. Hierbei werden vorteilhafterweise die Knochenstrukturen in einer Schädelbasisregion des Patienten voneinander abgegrenzt.

Selbstverständlich kann auch die Identifikation des Zielvolumens und/oder Risikovolumens im Verfahrensschritt 41-5-1 die Identifikation der Knochenstruktur und/oder die Abgrenzung zwischen grauer Gehirnsubstanz und weißer Gehirnsubstanz und/oder die Abgrenzung von Fettgewebe und Muskelgewebe umfassen.

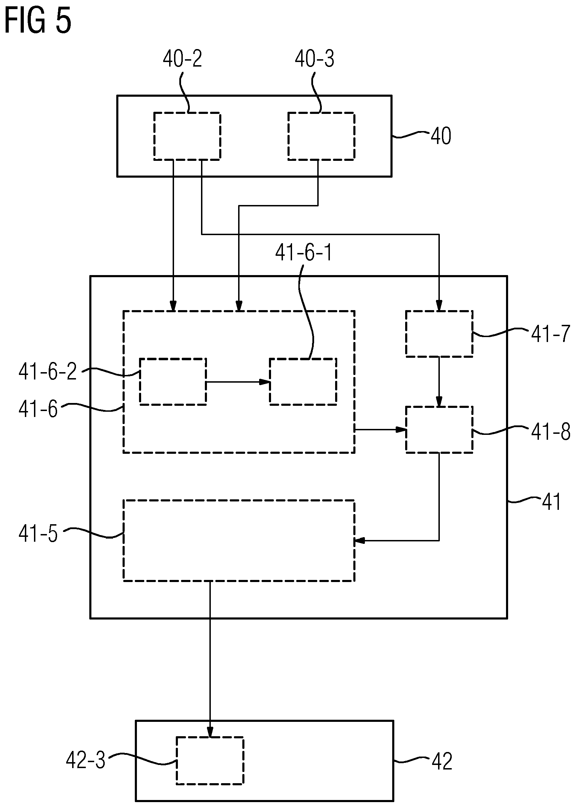

**Fig. 5** zeigt ein Ablaufdiagramm einer vierten Ausführungsform eines erfindungsgemäßen Verfahrens.

In Fig. 5 ist das Vorgehen dargestellt, dass das Erfassen der CT-Messdaten ein Erfassen eines ersten CT-Messdatensatzes in einem Verfahrensschritt 40-2 und ein Erfassen eines zweiten CT-Messdatensatzes in einem Verfahrensschritt 40-3 umfasst. Hierbei ist der erste CT-Messdatensatz mittels einer höheren Energieschwelle des quantenzählenden Röntgendetektors als der zweite CT-Messdatensatz akquiriert worden. Die Weiterverarbeitung der CT-Messdaten umfasst in einem Verfahrensschritt 41-6 ein Erstellen einer gewichteten Kombination des ersten CT-Messdatensatzes und des zweiten CT-Messdatensatzes, wobei kombinierte CT-Messdaten erzeugt werden. Derart erfolgt die Identifikation des Zielvolumens und/oder Risikovolumens in den kombinierten CT-Messdaten in einem Verfahrensschritt 41-5.

Hierbei ist in Fig. 5 das vorteilhafte Vorgehen gezeigt, dass die Weiterverarbeitung der CT-Messdaten in einem Verfahrensschritt 41-7 ein Extrahieren von Informationen für eine Strahlaufhärtungskorrektur aus dem ersten CT-Messdatensatz und in einem Verfahrensschritt 41-8 eine Strahlaufhärtungskorrektur der kombinierten CT-Messdaten basierend auf den extrahierten Informationen umfasst. Derart erfolgt in einem Verfahrensschritt 41-5 die Identifikation des Zielvolumens und/oder Risikovolumens in den durch die Strahlaufhärtungskorrektur korrigierten kombinierten CT-Messdaten.

Weiterhin erfolgt im Verfahrensschritt 41-6-1 das Erstellen der gewichteten Kombination des ersten CT-Messdatensatzes und des zweiten CT-Messdatensatzes unter Einsatz von räumlich variierenden Wichtungsfaktoren erfolgt. Hierfür umfasst im in Fig. 5 gezeigten Fall die Weiterverarbeitung der CT-Messdaten in einem Verfahrensschritt 41-6-2 zunächst eine grobe Identifikation des Zielvolumens und/oder Risikovolumens, wobei die räumlich variierenden Wichtungsfaktoren anhand des grob segmentierten Zielvolumens und/oder Risikovolumens definiert werden und anschließend eine feine Identifikation des Zielvolumens und/oder Risikovolumens in den kombinierten CT-Messdaten erfolgt.

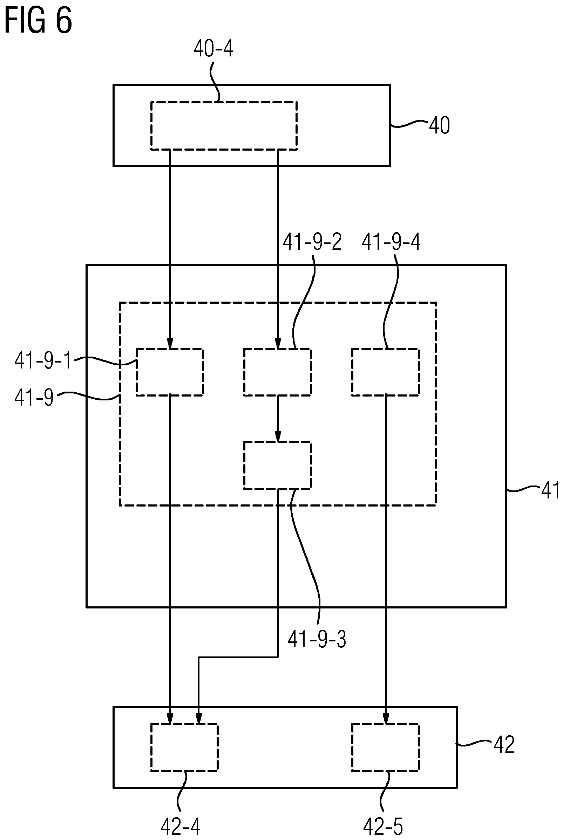

**Fig. 6** zeigt ein Ablaufdiagramm einer fünften Ausführungsform eines erfindungsgemäßen Verfahrens.

Das Erfassen der CT-Messdaten gemäß Fig. 6 umfasst in einem Verfahrensschritt 40-4 eine Akquisition von hoch-aufgelösten CT-Messdaten mittels des quantenzählenden Röntgendetektors, wobei ein hoch-auflösender Modus des quantenzählenden Röntgendetektors zur Akquisition der hoch-aufgelösten CT-Messdaten eingesetzt wird, wobei in dem hoch-aufgelösten Modus jeder Pixel des quantenzählenden Röntgendetektors separat gezählt wird.

Derart umfasst in einem Verfahrensschritt 41-9 die Weiterverarbeitung der CT-Messdaten ein Berechnen einer ortsaufgelösten Verteilung eines quantitativen Materialkoeffizienten aus den hoch-aufgelösten CT-Messdaten. Folglich umfasst das Bereitstellen der Ergebnisdaten ein Bereitstellen der ortsaufgelösten Verteilung des quantitativen Materialkoeffizienten an die Schnittstelle in einem Verfahrensschritt 42-4.

In Fig. 6 ist das vorteilhafte Vorgehen dargestellt, dass in einem Verfahrensschritt 41-9-1 die hoch-aufgelösten CT-Messdaten in das Berechnen der ortsaufgelösten Verteilung des quantitativen Materialkoeffizienten für ein Knochengewebe des Patienten in unveränderter Form eingehen und in einem Verfahrensschritt 41-9-3 die hoch-aufgelösten CT-Messdaten in das Berechnen der ortsaufgelösten Verteilung des quantitativen Materialkoeffizienten für ein Weichteilgewebe des Patienten in einer reduzierten Auflösung eingehen. Die reduzierte Auflösung der hoch-aufgelösten CT-Messdaten wird dabei in einem Verfahrensschritt 41-9-2 mittels Filterung der hoch-aufgelösten CT-Messdaten unter Verwendung eines Filterkernels erzeugt.

Auch ist in Fig. 6 zusätzlich das vorteilhafte Vorgehen dargestellt, dass in einem Verfahrensschritt 41-9-4 das Berechnen der ortsaufgelösten Verteilung des quantitativen Materialkoeffizienten ein Berechnen einer ortsaufgelösten Verteilung eines Bremsvermögens aus den hoch-aufgelösten CT-Messdaten umfasst und in einem Verfahrensschritt 42-5 das Bereitstellen der Ergebnisdaten ein Bereitstellen der ortsaufgelösten Verteilung des Bremsvermögens für eine Planung einer Partikel-Bestrahlung des Patienten umfasst.

Generell erfolgt das Berechnen der ortsaufgelösten Verteilung des quantitativen Materialkoeffizienten aus den hoch-aufgelösten CT-Messdaten vorteilhafterweise für zumindest eine der folgenden Körperregionen des Patienten:
- eine Augenregion des Patienten,
- eine durch einen Verlauf des Trigeminusnervs des Patienten beschriebene Körperregion,
- eine Schädelbasis des Patienten,
- eine Lungenregion des Patienten.

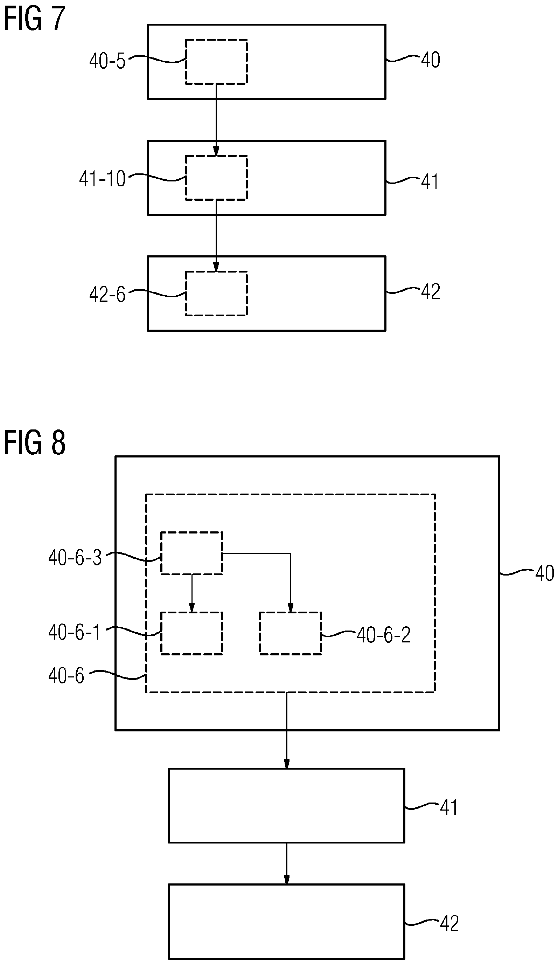

**Fig. 7** zeigt ein Ablaufdiagramm einer sechsten Ausführungsform eines erfindungsgemäßen Verfahrens.

In Fig. 7 ist das Vorgehen dargestellt, dass
- in einem Verfahrensschritt 40-5 das Erfassen der CT-Messdaten ein Erfassen von spektral-aufgelösten und zeitlich-aufgelösten CT-Messdaten, welche sich aus der Akquisition mittels des quantenzählenden Röntgendetektors ergeben, umfasst,
- in einem Verfahrensschritt 41-10 die Weiterverarbeitung der CT-Messdaten ein Berechnen einer ortsaufgelösten und zeitlich-aufgelösten Verteilung eines quantitativen Materialkoeffizienten aus den spektral-aufgelösten und zeitlich-aufgelösten CT-Messdaten umfasst und
- in einem Verfahrensschritt 42-6 das Bereitstellen der Ergebnisdaten ein Bereitstellen der ortsaufgelösten und zeitlich-aufgelösten Verteilung des quantitativen Materialkoeffizienten an die Schnittstelle umfasst.

Die spektral-aufgelösten und zeitlich-aufgelösten CT-Messdaten werden dabei vorteilhafterweise aus zumindest einer der folgenden Körperregionen des Patienten erfasst:
- eine Leberregion des Patienten,
- eine Pankreasregion des Patienten,
- einer Thoraxregion des Patienten.

**Fig. 8** zeigt ein Ablaufdiagramm einer siebten Ausführungsform eines erfindungsgemäßen Verfahrens.

Gemäß Fig. 8 umfasst das Erfassen der CT-Messdaten des Patienten in einem Verfahrensschritt 40-6 die Akquisition der CT-Messdaten mittels des CT-Geräts, welches den quantenzählenden Röntgendetektor aufweist, umfasst, wobei während der Akquisition der CT-Messdaten ein Detektorparameter des quantenzählenden Röntgendetektors verändert wird.

Insbesondere wird zumindest einer der folgenden Detektorparameter während der Akquisition der CT-Messdaten verändert:
- eine Energieschwelle des quantenzählenden Röntgendetektors,
- eine Auflösung des quantenzählenden Röntgendetektors bezüglich einer Kombination von Pixeln des quantenzählenden Röntgendetektors.

Im in Fig. 8 gezeigten Fall wird der Detektorparameter des quantenzählenden Röntgendetektors während der Akquisition der CT-Messdaten derart verändert wird, dass in einem Verfahrensschritt 40-6-1 für eine Akquisition der CT-Messdaten einer ersten Körperregion des Patienten eine erste Einstellung des Detektorparameters gesetzt wird und in einem Verfahrensschritt 40-6-2 für eine Akquisition der CT-Messdaten einer zweiten Körperregion des Patienten eine zweite Einstellung des Detektorparameters gesetzt wird. In einem Verfahrensschritt 40-6-3 werden die erste Körperregion und die zweite Körperregion vor der Akquisition der CT-Messdaten anhand von bereits vom Patienten vorliegenden medizinischen Bilddaten und/oder unter Verwendung einer Atlas-Datenbank festgelegt. Vorteilhafterweise wird während der Akquisition der CT-Messdaten der Detektorparameter des quantenzählenden Röntgendetektors derart verändert, dass für die Akquisition der CT-Messdaten aus der ersten Körperregion des Patienten eine höhere Auflösung des quantenzählenden Röntgendetektors gesetzt wird als für die Akquisition der CT-Messdaten aus der zweiten Körperregion des Patienten.

Die Körperregionen können gemäß den folgenden Eigenschaften ausgewählt werden. Hierbei ist eine beliebige Kombination der Eigenschaften denkbar:
- die erste Körperregion umfasst ein Zielvolumen der Bestrahlung umfasst und die zweite Körperregion umfasst eine außerhalb des Zielvolumens der Bestrahlung liegende Körperregion.
- die erste Körperregion ist derart ausgebildet, dass sie bei der Bestrahlung des Patienten in einem Strahlbereich positioniert ist, und die zweite Körperregion ist derart ausgebildet, dass sie bei der Bestrahlung des Patienten außerhalb eines Strahlbereichs positioniert ist.
- die erste Körperregion und die zweite Körperregion sind derart ausgebildet, dass bei der Bestrahlung des Patienten für die erste Körperregion eine höhere Strahlendosis vorgesehen ist als für die zweite Körperregion.

Alternativ oder zusätzlich ist auch das nicht dargestellte Vorgehen denkbar, dass der Detektorparameter des quantenzählenden Röntgendetektors während der Akquisition der CT-Messdaten derart verändert wird, dass für eine Akquisition der CT-Messdaten an einer ersten z-Position entlang einer Längsrichtung des Patienten eine erste Einstellung des Detektorparameters gesetzt wird und für eine Akquisition der CT-Messdaten an einer zweiten z-Position entlang der Längsrichtung des Patienten eine zweite Einstellung des Detektorparameters gesetzt wird.

Alternativ oder zusätzlich ist auch das nicht dargestellte Vorgehen denkbar, dass der Detektorparameter des quantenzählenden Röntgendetektors während der Akquisition der CT-Messdaten derart verändert wird, dass für eine Akquisition der CT-Messdaten bei einer ersten Rotationsposition eines Detektor-Strahler-Systems des CT-Geräts eine erste Einstellung des Detektorparameters gesetzt wird und für eine Akquisition der CT-Messdaten bei einer zweiten Rotationsposition des Detektor-Strahler-Systems des CT-Geräts eine zweite Einstellung des Detektorparameters gesetzt wird.

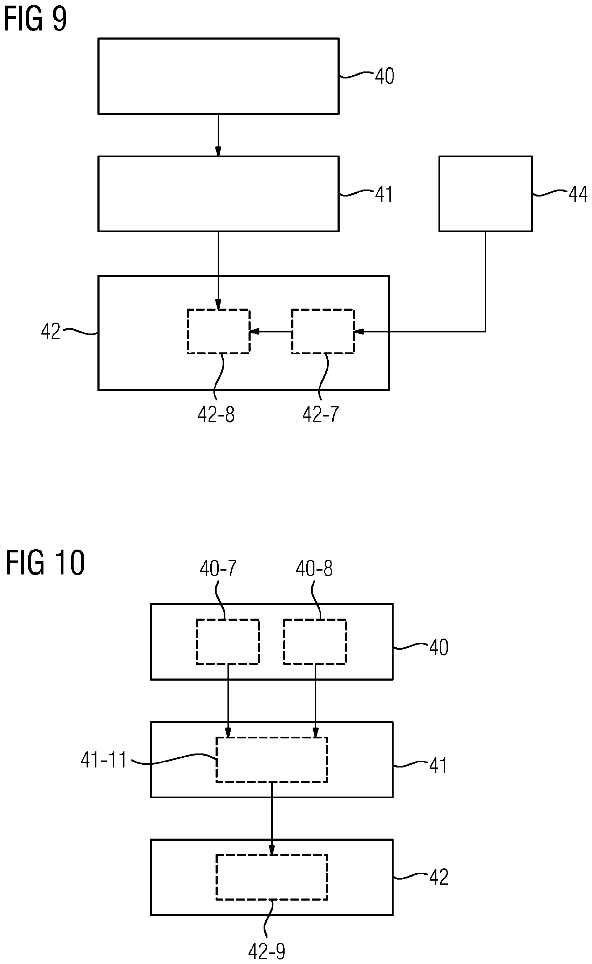

**Fig. 9** zeigt ein Ablaufdiagramm einer achten Ausführungsform eines erfindungsgemäßen Verfahrens.

Gemäß Fig. 9 erfasst in einem Verfahrensschritt 42-7 die Schnittstelle weitere Messdaten, welche mittels einer Bildgebungsvorrichtung eines für die Bestrahlung des Patienten eingesetzten Strahlentherapiegeräts erfasst werden, wobei in einem Verfahrensschritt 42-8 für die Planung der Bestrahlung des Patienten die Ergebnisdaten in Bezug zu den weiteren Messdaten gesetzt werden. Vorteilhafterweise weist die Bildgebungsvorrichtung des für die Bestrahlung des Patienten eingesetzten Strahlentherapiegeräts ebenfalls einen quantenzählenden Röntgendetektor auf, wobei die Ergebnisdaten unter Verwendung eines spezifische Informationsgehalts der CT-Messdaten und der weiteren CT-Messdaten, welcher sich aus der Verwendung des quantenzählenden Röntgendetektors bei der Akquisition der CT-Messdaten und der weiteren Messdaten ergibt, in Bezug zu den weiteren Messdaten gesetzt werden.

**Fig. 10** zeigt ein Ablaufdiagramm einer neunten Ausführungsform eines erfindungsgemäßen Verfahrens.

Gemäß Fig. 10 werden in einem Verfahrensschritt 40-7 ein erster CT-Messdatensatz und in einem Verfahrensschritt 40-8 ein zweiter CT-Messdatensatz erfasst. Beide CT-Messdatensätze sind zu unterschiedlichen Zeitpunkten erfasst worden. In einem Verfahrensschritt 41-11 werden die zumindest zwei CT-Messdatensätze unter Verwendung des spezifischen Informationsgehalts der CT-Messdaten, welcher sich aus der Verwendung des zumindest einen quantenzählenden Röntgendetektors bei der Akquisition der CT-Messdaten ergibt, registriert. In einem Verfahrensschritt 42-9 werden die registrierten zumindest zwei CT-Messdatensätze an die Schnittstelle bereitgestellt.

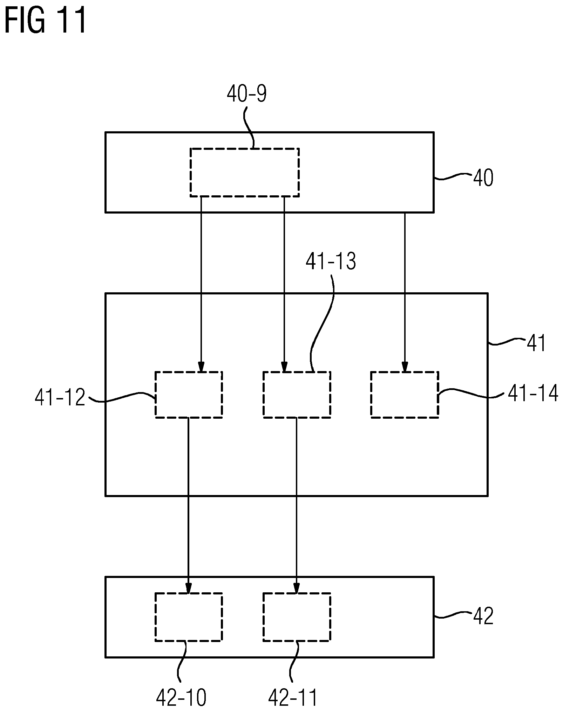

**Fig. 11** zeigt ein Ablaufdiagramm einer zehnten Ausführungsform eines erfindungsgemäßen Verfahrens.

Im in Fig. 11 gezeigten Fall ist in einem Verfahrensschritt 40-9 während der Akquisition der CT-Messdaten ein Kontrastmittel eingesetzt worden.

Gemäß einer ersten Anwendungsmöglichkeit umfasst die Weiterverarbeitung der CT-Messdaten in einem Verfahrensschritt 41-12 eine Materialdekomposition der CT-Messdaten in Gewebe, welches Kontrastmittel aufweist, und übriges Gewebe unter Verwendung des spezifischen Informationsgehalts der CT-Messdaten, welcher sich aus der Verwendung des zumindest einen quantenzählenden Röntgendetektors bei der Akquisition der CT-Messdaten ergibt. Die Materialdekomposition wird in einem Verfahrensschritt 42-10 an die Schnittstelle bereitgestellt.

Alternativ oder zusätzlich kann die zweite Anwendungsmöglichkeit eingesetzt werden, dass in einem Verfahrensschritt 41-13 die Weiterverarbeitung der CT-Messdaten eine Erstellung eines virtuellen nicht-kontrastierten CT-Bildes unter Verwendung des spezifischen Informationsgehalts der CT-Messdaten, welcher sich aus der Verwendung des zumindest einen quantenzählenden Röntgendetektors bei der Akquisition der CT-Messdaten ergibt, umfasst, wobei in einem Verfahrensschritt 42-11 das virtuelle nicht-kontrastierte CT-Bild an die Schnittstelle bereitgestellt wird.

Schließlich ist in Fig. 11 noch das Vorgehen dargestellt, dass die Weiterverarbeitung der CT-Messdaten in einem Verfahrensschritt 41-14 eine Erkennung und/oder Quantifizierung von Metall-Nanopartikeln, welche sich im Patienten zur Erhöhung eines Effekts der Bestrahlung des Patienten befinden, unter Verwendung des spezifischen Informationsgehalts der CT-Messdaten, welcher sich aus der Verwendung des zumindest einen quantenzählenden Röntgendetektors bei der Akquisition der CT-Messdaten ergibt, umfasst.

Die in Fig. 2-11 dargestellten Verfahrensschritte des erfindungsgemäßen Verfahrens werden von der Recheneinheit ausgeführt. Hierzu umfasst die Recheneinheit erforderliche Software und/oder Computerprogramme, die in einer Speichereinheit der Recheneinheit gespeichert sind. Die Software und/oder Computerprogramme umfassen Programmmittel, die dazu ausgelegt sind, das erfindungsgemäße Verfahren auszuführen, wenn das Computerprogramm und/oder die Software in der Recheneinheit mittels einer Prozessoreinheit der Recheneinheit ausgeführt wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zum Bereitstellen von Ergebnisdaten, welche geeignet für einen Einsatz in einer Planung einer Bestrahlung eines Patienten sind, umfassend folgende Verfahrensschritte:
- Erfassen von CT-Messdaten des Patienten, welche mittels eines CT-Geräts, welches einen quantenzählenden Röntgendetektor aufweist, akquiriert worden sind,
- Weiterverarbeitung der CT-Messdaten, wobei bei der Weiterverarbeitung der CT-Messdaten ein spezifischer Informationsgehalt der CT-Messdaten, welcher sich aus der Verwendung des quantenzählenden Röntgendetektors bei der Akquisition der CT-Messdaten ergibt, berücksichtigt wird, wobei bei der Weiterverarbeitung der CT-Messdaten Ergebnisdaten erzeugt werden, welche geeignet für den Einsatz in der Planung der Bestrahlung des Patienten sind,
- Bereitstellen der Ergebnisdaten an eine Schnittstelle, so dass die Ergebnisdaten für die Planung der Bestrahlung des Patienten verwendet werden können.

2. Verfahren nach Anspruch 1, wobei das Verfahren den folgenden zusätzlichen Verfahrensschritt umfasst:
- Durchführen der Planung der Bestrahlung des Patienten, wobei die Ergebnisdaten von der Schnittstelle abgerufen werden und bei der Planung der Bestrahlung des Patienten eingesetzt werden.

3. Verfahren nach einem der vorhergehenden Ansprüche, wobei
- das Erfassen der CT-Messdaten ein Erfassen von spektral-aufgelösten CT-Messdaten, welche sich aus der Akquisition mittels des quantenzählenden Röntgendetektors ergeben, umfasst,
- die Weiterverarbeitung der CT-Messdaten ein Berechnen einer ortsaufgelösten Verteilung eines quantitativen Materialkoeffizienten aus den spektral-aufgelösten CT-Messdaten umfasst und
- das Bereitstellen der Ergebnisdaten ein Bereitstellen der ortsaufgelösten Verteilung des quantitativen Materialkoeffizienten an die Schnittstelle umfasst.

4. Verfahren nach Anspruch 3, wobei die Weiterverarbeitung der CT-Messdaten ein Berechnen einer ersten ortsaufgelösten Verteilung eines ersten quantitativen Materialkoeffizienten und einer zweiten ortsaufgelösten Verteilung eines zweiten quantitativen Materialkoeffizienten aus den spektral-aufgelösten CT-Messdaten umfasst.

5. Verfahren nach Anspruch 4 wobei das Bereitstellen der Ergebnisdaten ein Bereitstellen der ersten ortsaufgelösten Verteilung des ersten quantitativen Materialkoeffizienten für einen ersten Dosisberechnungsalgorithmus und ein Bereitstellen der zweiten ortsaufgelösten Verteilung des zweiten quantitativen Materialkoeffizienten für einen zweiten Dosisberechnungsalgorithmus umfasst.

6. Verfahren nach einem der Ansprüche 4-5, wobei der erste quantitative Materialkoeffizient eine Elektronendichte und der zweite quantitative Materialkoeffizient eine effektive Kernladungszahl ist.

7. Verfahren nach einem der Ansprüche 4-5, wobei der erste quantitative Materialkoeffizient und der zweite quantitative Materialkoeffizient auf zwei voneinander linear-unabhängigen Basismaterialien basieren.

8. Verfahren nach einem der Ansprüche 4-7, wobei die Weiterverarbeitung der CT-Messdaten eine getrennte Weiterverarbeitung der ersten ortsaufgelösten Verteilung des ersten quantitativen Materialkoeffizienten und der zweiten ortsaufgelösten Verteilung des zweiten quantitativen Materialkoeffizienten und eine Erzeugung der Ergebnisdaten mittels einer Zusammenführung der bei der getrennten Weiterverarbeitung erzeugten Teil-Ergebnisdaten umfasst.

9. Verfahren nach einem der Ansprüche 3-8, wobei das Erfassen der CT-Messdaten ein Laden von zuvor akquirierten spektral-aufgelösten CT-Messdaten aus einer Datenbank zur Berechnung der ortsaufgelösten Verteilung des quantitativen Materialkoeffizienten umfasst.

10. Verfahren nach einem der Ansprüche 3-9, wobei die Weiterverarbeitung der CT-Messdaten zusätzlich zum Berechnen der ortsaufgelösten Verteilung des quantitativen Materialkoeffizienten aus den spektral-aufgelösten CT-Messdaten eine Identifikation eines Zielvolumens und/oder Risikovolumens in den spektral-aufgelösten CT-Messdaten umfasst.

11. Verfahren nach Anspruch 10, wobei die spektral-aufgelösten CT-Messdaten mittels derartiger Aufnahmeparameter des quantenzählenden Röntgendetektors akquiriert worden sind, dass die spektral-aufgelösten CT-Messdaten einen besonders geeigneten Kontrast zwischen verschiedenen Gewebetypen für die Identifikation des Zielvolumens und/oder Risikovolumens aufweisen.

12. Verfahren nach einem der Ansprüche 3-11, wobei die spektral-aufgelösten CT-Messdaten ein Messfeld abdecken, welches in einer axialen Messschicht sowohl den gesamten Körper des Patienten, als auch für die Lagerung des Patienten verwendete Positionierungshilfen umfasst.

13. Verfahren nach einem der Ansprüche 3-12, wobei die Weiterverarbeitung der CT-Messdaten eine Strahlaufhärtungskorrektur der spektral-aufgelösten CT-Messdaten unter Verwendung von in den spektral-aufgelösten CT-Messdaten enthaltenen spektralen Informationen umfasst, wobei das Berechnen der ortsaufgelösten Verteilung des quantitativen Materialkoeffizienten aus den durch die Strahlaufhärtungskorrektur korrigierten spektral-aufgelösten CT-Messdaten erfolgt.

14. Verfahren nach einem der vorhergehenden Ansprüche, wobei die Weiterverarbeitung der CT-Messdaten eine Identifikation eines Zielvolumens und/oder Risikovolumens in den CT-Messdaten umfasst, wobei das Bereitstellen der Ergebnisdaten ein Bereitstellen des identifizierten Zielvolumens und/oder Risikovolumens an die Schnittstelle umfasst.

15. Verfahren nach Anspruch 14, wobei die Identifikation des Zielvolumens und/oder Risikovolumens eine Abgrenzung eines Gewebes des Zielvolumens und/oder Risikovolumens von umliegendem Gewebe umfasst, wobei die Abgrenzung des Gewebes des Zielvolumens und/oder Risikovolumens vom umliegenden Gewebe unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

16. Verfahren nach einem der Ansprüche 14-15, wobei die Identifikation des Zielvolumens und/oder Risikovolumens eine Abgrenzung von Fettgewebe und Muskelgewebe umfasst, wobei die Abgrenzung des Fettgewebes und Muskelgewebes unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

17. Verfahren nach einem der Ansprüche 14-16, wobei die Identifikation des Zielvolumens und/oder Risikovolumens eine Abgrenzung von grauer Gehirnsubstanz und weißer Gehirnsubstanz umfasst, wobei die Abgrenzung der grauen Gehirnsubstanz und weißen Gehirnsubstanz unter Verwendung des spezifischen Informationsgehalt der CT-Messdaten, welcher sich aus der Verwendung des zumindest einen quantenzählenden Röntgendetektors bei der Akquisition der CT-Messdaten ergibt, erfolgt.

18. Verfahren nach einem der Ansprüche 14-17, wobei
- das Erfassen der CT-Messdaten ein Erfassen von spektral-aufgelösten CT-Messdaten, welche sich aus der Akquisition mittels des quantenzählenden Röntgendetektors ergeben, umfasst,
- die Weiterverarbeitung der CT-Messdaten eine Strahlaufhärtungskorrektur der spektral-aufgelösten CT-Messdaten unter Verwendung von in den spektral-aufgelösten CT-Messdaten enthaltenen spektralen Informationen umfasst und
- die Identifikation des Zielvolumens und/oder Risikovolumens eine Abgrenzung von Knochenstrukturen in den durch die Strahlaufhärtungskorrektur korrigierten spektral-aufgelösten CT-Messdaten umfasst.

19. Verfahren nach Anspruch 18, wobei die Knochenstrukturen in einer Schädelbasisregion des Patienten voneinander abgegrenzt werden.

20. Verfahren nach einem der Ansprüche 14-19, wobei
- die CT-Messdaten einen ersten CT-Messdatensatz und einen zweiten CT-Messdatensatz umfassen, wobei der erste CT-Messdatensatz mittels einer höheren Energieschwelle des quantenzählenden Röntgendetektors als der zweite CT-Messdatensatz akquiriert worden ist,
- die Weiterverarbeitung der CT-Messdaten ein Erstellen einer gewichteten Kombination des ersten CT-Messdatensatzes und des zweiten CT-Messdatensatzes umfasst, wobei kombinierte CT-Messdaten erzeugt werden und
- die Identifikation des Zielvolumens und/oder Risikovolumens in den kombinierten CT-Messdaten erfolgt.

21. Verfahren nach Anspruch 20, wobei
- die Weiterverarbeitung der CT-Messdaten ein Extrahieren von Informationen für eine Strahlaufhärtungskorrektur aus dem ersten CT-Messdatensatz und eine Strahlaufhärtungskorrektur der kombinierten CT-Messdaten basierend auf den extrahierten Informationen umfasst, wobei
- die Identifikation des Zielvolumens und/oder Risikovolumens in den durch die Strahlaufhärtungskorrektur korrigierten kombinierten CT-Messdaten erfolgt.

22. Verfahren nach einem der Ansprüche 20-21, wobei das Erstellen der gewichteten Kombination des ersten CT-Messdatensatzes und des zweiten CT-Messdatensatzes unter Einsatz von räumlich variierenden Wichtungsfaktoren erfolgt.

23. Verfahren nach Anspruch 22, wobei die Weiterverarbeitung der CT-Messdaten zunächst eine grobe Identifikation des Zielvolumens und/oder Risikovolumens umfasst, wobei die räumlich variierenden Wichtungsfaktoren anhand des grob segmentierten Zielvolumens und/oder Risikovolumens definiert werden und anschließend eine feine Identifikation des Zielvolumens und/oder Risikovolumens in den kombinierten CT-Messdaten erfolgt.

24. Verfahren nach einem der vorhergehenden Ansprüche, wobei das Erfassen der CT-Messdaten eine Akquisition von hoch-aufgelösten CT-Messdaten mittels des quantenzählenden Röntgendetektors umfasst, wobei ein hoch-auflösender Modus des quantenzählenden Röntgendetektors zur Akquisition der hoch-aufgelösten CT-Messdaten eingesetzt wird, wobei in dem hoch-aufgelösten Modus jeder Pixel des quantenzählenden Röntgendetektors separat gezählt wird.

25. Verfahren nach Anspruch 24, wobei
- die Weiterverarbeitung der CT-Messdaten ein Berechnen einer ortsaufgelösten Verteilung eines quantitativen Materialkoeffizienten aus den hoch-aufgelösten CT-Messdaten umfasst und
- das Bereitstellen der Ergebnisdaten ein Bereitstellen der ortsaufgelösten Verteilung des quantitativen Materialkoeffizienten an die Schnittstelle umfasst.

26. Verfahren nach Anspruch 25, wobei
- die hoch-aufgelösten CT-Messdaten in das Berechnen der ortsaufgelösten Verteilung des quantitativen Materialkoeffizienten für ein Knochengewebe des Patienten in unveränderter Form eingehen und
- die hoch-aufgelösten CT-Messdaten in das Berechnen der ortsaufgelösten Verteilung des quantitativen Materialkoeffizienten für ein Weichteilgewebe des Patienten in einer reduzierten Auflösung eingehen.

27. Verfahren nach Anspruch 26, wobei die reduzierte Auflösung der hoch-aufgelösten CT-Messdaten mittels Filterung der hoch-aufgelösten CT-Messdaten unter Verwendung eines Filterkernels erzeugt wird.

28. Verfahren nach einem der Ansprüche 25-27, wobei
- das Berechnen der ortsaufgelösten Verteilung des quantitativen Materialkoeffizienten ein Berechnen einer ortsaufgelösten Verteilung eines Bremsvermögens aus den hoch-aufgelösten CT-Messdaten umfasst und
- das Bereitstellen der Ergebnisdaten ein Bereitstellen der ortsaufgelösten Verteilung des Bremsvermögens für eine Planung einer Partikel-Bestrahlung des Patienten umfasst.

29. Verfahren nach einem der Ansprüche 25-28, wobei das Berechnen der ortsaufgelösten Verteilung des quantitativen Materialkoeffizienten aus den hoch-aufgelösten CT-Messdaten für zumindest eine der folgenden Körperregionen des Patienten erfolgt:
- eine Augenregion des Patienten,
- eine durch einen Verlauf des Trigeminusnervs des Patienten beschriebene Körperregion,
- eine Schädelbasis des Patienten,
- eine Lungenregion des Patienten.

30. Verfahren nach einem der vorhergehenden Ansprüche, wobei
- das Erfassen der CT-Messdaten ein Erfassen von spektral-aufgelösten und zeitlich-aufgelösten CT-Messdaten, welche sich aus der Akquisition mittels des quantenzählenden Röntgendetektors ergeben, umfasst,
- die Weiterverarbeitung der CT-Messdaten ein Berechnen einer ortsaufgelösten und zeitlich-aufgelösten Verteilung eines quantitativen Materialkoeffizienten aus den spektral-aufgelösten und zeitlich-aufgelösten CT-Messdaten umfasst und
- das Bereitstellen der Ergebnisdaten ein Bereitstellen der ortsaufgelösten und zeitlich-aufgelösten Verteilung des quantitativen Materialkoeffizienten an die Schnittstelle umfasst.

31. Verfahren nach Anspruch 30, wobei die spektral-aufgelösten und zeitlich-aufgelösten CT-Messdaten aus zumindest einer der folgenden Körperregionen des Patienten erfasst werden:
- eine Leberregion des Patienten,
- eine Pankreasregion des Patienten,
- einer Thoraxregion des Patienten.

32. Verfahren nach einem der vorhergehenden Ansprüche, wobei das Erfassen der CT-Messdaten des Patienten die Akquisition der CT-Messdaten mittels des CT-Geräts, welches den quantenzählenden Röntgendetektor aufweist, umfasst, wobei während der Akquisition der CT-Messdaten ein Detektorparameter des quantenzählenden Röntgendetektors verändert wird.

33. Verfahren nach Anspruch 32, wobei während der Akquisition der CT-Messdaten eine Energieschwelle des quantenzählenden Röntgendetektors verändert wird.

34. Verfahren nach einem der Ansprüche 32-33, wobei während der Akquisition der CT-Messdaten eine Auflösung des quantenzählenden Röntgendetektors bezüglich einer Kombination von Pixeln des quantenzählenden Röntgendetektors verändert wird.

35. Verfahren nach einem der Ansprüche 32-34, wobei der Detektorparameter des quantenzählenden Röntgendetektors während der Akquisition der CT-Messdaten derart verändert wird, dass für eine Akquisition der CT-Messdaten an einer ersten z-Position entlang einer Längsrichtung des Patienten eine erste Einstellung des Detektorparameters gesetzt wird und für eine Akquisition der CT-Messdaten an einer zweiten z-Position entlang der Längsrichtung des Patienten eine zweite Einstellung des Detektorparameters gesetzt wird.

36. Verfahren nach einem der Ansprüche 32-35, wobei der Detektorparameter des quantenzählenden Röntgendetektors während der Akquisition der CT-Messdaten derart verändert wird, dass für eine Akquisition der CT-Messdaten bei einer ersten Rotationsposition eines Detektor-Strahler-Systems des CT-Geräts eine erste Einstellung des Detektorparameters gesetzt wird und für eine Akquisition der CT-Messdaten bei einer zweiten Rotationsposition des Detektor-Strahler-Systems des CT-Geräts eine zweite Einstellung des Detektorparameters gesetzt wird.

37. Verfahren nach einem der vorhergehenden Ansprüche 32-36, wobei der Detektorparameter des quantenzählenden Röntgendetektors während der Akquisition der CT-Messdaten derart verändert wird, dass für eine Akquisition der CT-Messdaten einer ersten Körperregion des Patienten eine erste Einstellung des Detektorparameters gesetzt wird und für eine Akquisition der CT-Messdaten einer zweiten Körperregion des Patienten eine zweite Einstellung des Detektorparameters gesetzt wird.

38. Verfahren nach Anspruch 37, wobei die erste Körperregion und die zweite Körperregion vor der Akquisition der CT-Messdaten anhand von bereits vom Patienten vorliegenden medizinischen Bilddaten und/oder unter Verwendung einer Atlas-Datenbank festgelegt werden.

39. Verfahren nach einem der Ansprüche 37-38, wobei die erste Körperregion ein Zielvolumen der Bestrahlung umfasst und die zweite Körperregion eine außerhalb des Zielvolumens der Bestrahlung liegende Körperregion umfasst.

40. Verfahren nach einem der Ansprüche 37-39, wobei die erste Körperregion derart ausgebildet ist, dass sie bei der Bestrahlung des Patienten in einem Strahlbereich positioniert ist, und die zweite Körperregion derart ausgebildet ist, dass sie bei der Bestrahlung des Patienten außerhalb eines Strahlbereichs positioniert ist.

41. Verfahren nach einem der Ansprüche 37-40, wobei die erste Körperregion und die zweite Körperregion derart ausgebildet sind, dass bei der Bestrahlung des Patienten für die erste Körperregion eine höhere Strahlendosis vorgesehen ist als für die zweite Körperregion.

42. Verfahren nach einem der Ansprüche 37-41, wobei während der Akquisition der CT-Messdaten der Detektorparameter des quantenzählenden Röntgendetektors derart verändert wird, dass für die Akquisition der CT-Messdaten aus der ersten Körperregion des Patienten eine höhere Auflösung des quantenzählenden Röntgendetektors gesetzt wird als für die Akquisition der CT-Messdaten aus der zweiten Körperregion des Patienten.

43. Verfahren nach einem der vorhergehenden Ansprüche, wobei die Schnittstelle weitere Messdaten erfasst, welche mittels einer Bildgebungsvorrichtung eines für die Bestrahlung des Patienten eingesetzten Strahlentherapiegeräts erfasst werden, wobei für die Planung der Bestrahlung des Patienten die Ergebnisdaten in Bezug zu den weiteren Messdaten gesetzt werden.

44. Verfahren nach Anspruch 43, wobei die Bildgebungsvorrichtung des für die Bestrahlung des Patienten eingesetzten Strahlentherapiegeräts ebenfalls einen quantenzählenden Röntgendetektor aufweist, wobei die Ergebnisdaten unter Verwendung eines spezifische Informationsgehalts der CT-Messdaten und der weiteren CT-Messdaten, welcher sich aus der Verwendung des quantenzählenden Röntgendetektors bei der Akquisition der CT-Messdaten und der weiteren Messdaten ergibt, in Bezug zu den weiteren Messdaten gesetzt werden.

45. Verfahren nach einem der vorhergehenden Ansprüche, wobei die CT-Messdaten zumindest zwei CT-Messdatensätze umfasst, welche zu unterschiedlichen Zeitpunkten erfasst worden sind, wobei die zumindest zwei CT-Messdatensätze unter Verwendung des spezifischen Informationsgehalts der CT-Messdaten, welcher sich aus der Verwendung des zumindest einen quantenzählenden Röntgendetektors bei der Akquisition der CT-Messdaten ergibt, registriert werden, wobei die registrierten zumindest zwei CT-Messdatensätze an die Schnittstelle bereitgestellt werden.

46. Verfahren nach einem der vorhergehenden Ansprüche, wobei während der Akquisition der CT-Messdaten ein Kontrastmittel eingesetzt worden ist, wobei die Weiterverarbeitung der CT-Messdaten eine Materialdekomposition der CT-Messdaten in Gewebe, welches Kontrastmittel aufweist, und übriges Gewebe unter Verwendung des spezifischen Informationsgehalts der CT-Messdaten, welcher sich aus der Verwendung des zumindest einen quantenzählenden Röntgendetektors bei der Akquisition der CT-Messdaten ergibt, umfasst.

47. Verfahren nach einem der vorhergehenden Ansprüche, wobei während der Akquisition der CT-Messdaten ein Kontrastmittel eingesetzt worden ist, wobei die Weiterverarbeitung der CT-Messdaten eine Erstellung eines virtuellen nicht-kontrastierten CT-Bildes unter Verwendung des spezifischen Informationsgehalts der CT-Messdaten, welcher sich aus der Verwendung des zumindest einen quantenzählenden Röntgendetektors bei der Akquisition der CT-Messdaten ergibt, umfasst.

48. Recheneinheit, umfassend zumindest ein Rechenmodul, wobei die Recheneinheit zum Ausführen eines Verfahrens nach einem der vorhergehenden Ansprüche ausgebildet ist.

49. CT-Gerät mit einer Recheneinheit nach Anspruch 48.

50. Computerprogrammprodukt, welches direkt in einen Speicher einer programmierbaren Recheneinheit ladbar ist, mit Programmcode-Mitteln, um ein Verfahren nach einem der Ansprüche 1-47 auszuführen, wenn das Computerprogrammprodukt in der Recheneinheit ausgeführt wird.

## Claims

1. Method for the provision of result data which is suitable for use in planning an irradiation of a patient, comprising the following steps:
- The acquisition of CT measurement data of the patient which has been acquired by means of a CT device having a quantum counting X-ray detector,
- The processing of the CT measurement data, wherein during the processing of the CT measurement data a specific information content of the CT measurement data resulting from the use of the quantum counting X-ray detector in the acquisition of the CT measurement data is taken into account, wherein result data is generated during the processing of the CT measurement data which is suitable for use in planning of the irradiation of the patient,
- The provision of the result data to an interface such that the result data can be used for the planning of the irradiation of the patient.

2. Method according to claim 1, wherein the method comprises the following additional step:
- The performance of the planning of the irradiation of the patient, wherein the result data is retrieved from the interface and is used for the planning of the irradiation of the patient.

3. Method according to one of the preceding claims, wherein
- The acquisition of the CT measurement data comprises the acquisition of spectrally resolved CT measurement data resulting from acquisition by means of the quantum counting X-ray detector,
- The processing of the CT measurement data comprises a calculation of a spatially resolved distribution of a quantitative material coefficient from the spectrally resolved CT measurement data and
- The provision of the result data comprises the provision of the spatially resolved distribution of the quantitative material coefficient to the interface.

4. Method according to claim 3, wherein the processing of the CT measurement data comprises a calculation of a first spatially resolved distribution of a first quantitative material coefficient and a second spatially resolved distribution of a second quantitative material coefficient from the spectrally resolved CT measurement data.

5. Method according to claim 4, wherein the provision of the result data comprises the provision of the first spatially resolved distribution of the first quantitative material coefficient for a first dose calculation algorithm and the provision of the second spatially resolved distribution of the second quantitative material coefficient for a second dose calculation algorithm.

6. Method according to one of claims 4-5, wherein the first quantitative material coefficient is an electron density and the second quantitative material coefficient an effective atomic number.

7. Method according to one of claims 4-5, wherein the first quantitative material coefficient and the second quantitative material coefficient are based on two base materials linearly independent of each other.

8. Method according to one of claims 4-7, wherein the processing of the CT measurement data comprises a separate processing of the first spatially resolved distribution of the first quantitative material coefficient and the second spatially resolved distribution of the second quantitative material coefficient and a generation of the result data by combining the partial result data generated during the separate processing.

9. Method according to one of claims 3-8, wherein the acquisition of the CT measurement data comprises the loading of previously acquired spectrally resolved CT measurement data from a database for the calculation of the spatially resolved distribution of the quantitative material coefficient.

10. Method according to one of claims 3-9, wherein, in addition to the calculation of the spatially resolved distribution of the quantitative material coefficient from the spectrally resolved CT measurement data, the processing of the CT measurement data comprises the identification of a target volume and/or risk volume in the spectrally resolved CT measurement data.

11. Method according to claim 10, wherein the spectrally resolved CT measurement data has been acquired by means of such acquisition parameters of the quantum counting X-ray detector that the spectrally resolved CT measurement data displays a particularly suitable contrast between different types of tissue for the identification of the target volume and/or risk volume.

12. Method according to one of claims 3-11, wherein the spectrally resolved CT measurement data covers a field of view which comprises both the entire body of the patient and positioning aids used for the support of the patient in an axial measuring layer.

13. Method according to one of claims 3-12, wherein the processing of the CT measurement data comprises a beam hardening correction of the spectrally resolved CT measurement data using spectral information contained in the spectrally resolved CT measurement data, wherein the spatially resolved distribution of the quantitative material coefficient is calculated from the spectrally resolved CT measurement data corrected by the beam hardening correction.

14. Method according to one of the preceding claims, wherein the processing of the CT measurement data comprises the identification of a target volume and/or risk volume in the CT measurement data, wherein the provision of the result data comprises the provision of the identified target volume and/or risk volume to the interface.

15. Method according to claim 14, wherein the identification of the target volume and/or risk volume comprises a differentiation of a tissue of the target volume and/or risk volume from the surrounding tissue, wherein the differentiation of the tissue of the target volume and/or risk volume from the surrounding tissue takes place using the specific information content of the CT measurement data resulting from the use of the at least one quantum counting X-ray detector in the acquisition of the CT measurement data.

16. Method according to one of claims 14-15, wherein the identification of the target volume and/or risk volume comprises a differentiation of adipose tissue and muscle tissue, wherein the differentiation of the adipose tissue and muscle tissue takes place using the specific information content of the CT measurement data resulting from the use of the at least one quantum counting X-ray detector in the acquisition of the CT measurement data.

17. Method according to one of claims 14-16, wherein the identification of the target volume and/or risk volume comprises a differentiation of gray matter and white matter, wherein the differentiation of gray matter and white matter takes place using the specific information content of the CT measurement data resulting from the use of the at least one quantum counting X-ray detector in the acquisition of the CT measurement data.

18. Method according to one of claims 14-17, wherein
- The acquisition of the CT measurement data comprises the acquisition of spectrally resolved CT measurement data resulting from acquisition by means of the quantum counting X-ray detector,
- The processing of the CT measurement data comprises a beam hardening correction of the spectrally resolved CT measurement data using spectral information contained in the spectrally resolved CT measurement data and
- The identification of the target volume and/or risk volume comprises a differentiation of bone structures in the spectrally resolved CT measurement data corrected by the beam hardening correction.

19. Method according to claim 18, wherein the bone structures in a skull base region of the patient are differentiated from each other.

20. Method according to one of claims 14-19, wherein
- The CT measurement data comprises a first CT measurement data set and a second CT measurement data set, wherein the first CT measurement data set has been acquired by means of a higher energy threshold of the quantum counting X-ray detector than the second CT measurement data set,
- The processing of the CT measurement data comprises the creation of a weighted combination of the first CT measurement data set and of the second CT measurement data set, wherein combined CT measurement data is generated and
- The identification of the target volume and/or risk volume takes place in the combined CT measurement data.

21. Method according to claim 20, wherein
- The processing of the CT measurement data comprises the extraction of information for a beam hardening correction from the first CT measurement data set and a beam hardening correction of the combined CT measurement data based on the extracted information, wherein
- The identification of the target volume and/or risk volume takes place in the combined CT measurement data corrected by the beam hardening correction.

22. Method according to one of claims 20-21, wherein the creation of the weighted combination of the first CT measurement data set and of the second CT measurement data set takes place using spatially varying weighting factors.

23. Method according to claim 22, wherein the processing of the CT measurement data first comprises a rough identification of the target volume and/or risk volume, wherein the spatially varying weighting factors are defined by means of the roughly segmented target volume and/or risk volume and then a fine identification of the target volume and/or risk volume takes place in the combined CT measurement data.

24. Method according to one of the preceding claims, wherein the acquisition of the CT measurement data comprises the acquisition of high-resolution CT measurement data by means of the quantum counting X-ray detector, wherein a high-resolution mode of the quantum counting X-ray detector is used for the acquisition of the high-resolution CT measurement data, wherein each pixel of the quantum counting X-ray detector is counted separately in the high-resolution mode.

25. Method according to claim 24, wherein
- The processing of the CT measurement data comprises a calculation of a spatially resolved distribution of a quantitative material coefficient from the high-resolution CT measurement data and
- The provision of the result data comprises the provision of the spatially resolved distribution of the quantitative material coefficient to the interface.

26. Method according to claim 25, wherein
- The high-resolution CT measurement data is included in the calculation of the spatially resolved distribution of the quantitative material coefficient for a bone tissue of the patient in unaltered form and
- The high-resolution CT measurement data is included in the calculation of the spatially resolved distribution of the quantitative material coefficient for a soft tissue of the patient in a reduced resolution.

27. Method according to claim 26, wherein the reduced resolution of the high-resolution CT measurement data is generated by filtering the high-resolution CT measurement data using a filter kernel.

28. Method according to one of claims 25-27, wherein
- The calculation of the spatially resolved distribution of the quantitative material coefficient comprises a calculation of a spatially resolved distribution of a stopping power from the high-resolution CT measurement data and
- The provision of the result data comprises the provision of the spatially resolved distribution of the stopping power for the planning of a particle irradiation of the patient.

29. Method according to one of claims 25-28, wherein the spatially resolved distribution of the quantitative material coefficient is calculated from the high-resolution CT measurement data for at least one of the following body regions of the patient:
- An eye region of the patient,
- A body region described by a course of the trigeminal nerve of the patient,
- The skull base of the patient,
- A lung region of the patient.

30. Method according to one of the preceding claims, wherein
- The acquisition of the CT measurement data comprises the acquisition of spectrally resolved and temporally resolved CT measurement data resulting from acquisition by means of the quantum counting X-ray detector,
- The processing of the CT measurement data comprises the calculation of a spatially resolved and temporally resolved distribution of a quantitative material coefficient from the spectrally resolved and temporally resolved CT measurement data and
- The provision of the result data comprises the provision of the spatially resolved and temporally resolved distribution of the quantitative material coefficient to the interface.

31. Method according to claim 30, wherein the spectrally resolved and temporally resolved CT measurement data is acquired from at least one of the following body regions of the patient:
- A liver region of the patient,
- A pancreas region of the patient,
- A thorax region of the patient.

32. Method according to one of the preceding claims, wherein the acquisition of the CT measurement data of the patient comprises the acquisition of the CT measurement data by means of the CT device having the quantum counting X-ray detector, wherein during the acquisition of the CT measurement data a detector parameter of the quantum counting X-ray detector is changed.

33. Method according to claim 32, wherein during the acquisition of the CT measurement data an energy threshold of the quantum counting X-ray detector is changed.

34. Method according to one of claims 32-33, wherein during the acquisition of the CT measurement data a resolution of the quantum counting X-ray detector is changed with regard to a combination of pixels of the quantum counting X-ray detector.

35. Method according to one of claims 32-34, wherein the detector parameter of the quantum counting X-ray detector is changed during the acquisition of the CT measurement data such that a first setting of the detector parameter is set for the acquisition of the CT measurement data at a first z-position along a longitudinal direction of the patient, and a second setting of the detector parameter is set for an acquisition of the CT measurement data at a second z-position along the longitudinal direction of the patient.

36. Method according to one of claims 32-35, wherein the detector parameter of the quantum counting X-ray detector is changed during the acquisition of the CT measurement data such that a first setting of the detector parameter is set for the acquisition of the CT measurement data in a first rotational position of a detector-emitter system of the CT device and a second setting of the detector parameter is set for the acquisition of the CT measurement data in a second rotational position of the detector-emitter system of the CT device.

37. Method according to one of the preceding claims 32-36, wherein the detector parameter of the quantum counting X-ray detector is changed during the acquisition of the CT measurement data such that a first setting of the detector parameter is set for the acquisition of the CT measurement data of a first body region of the patient and a second setting of the detector parameter is set for the acquisition of the CT measurement data of a second body region of the patient.

38. Method according to claim 37, wherein the first body region and the second body region are established before the acquisition of the CT measurement data by means of existing medical image data of the patient and/or by using an Atlas database.

39. Method according to one of claims 37-38, wherein the first body region comprises a target volume of the irradiation and the second body region comprises a body region outside the target volume of the irradiation.

40. Method according to one of claims 37-39, wherein the first body region is embodied such that during the irradiation of the patient it is positioned in a beam region, and the second body region is embodied such that during the irradiation of the patient it is positioned outside a beam region.

41. Method according to one of claims 37-40, wherein the first body region and the second body region are embodied such that during the irradiation of the patient a higher radiation dose is provided for the first body region than for the second body region.

42. Method according to one of claims 37-41, wherein during the acquisition of the CT measurement data the detector parameter of the quantum counting X-ray detector is changed such that a higher resolution of the quantum counting X-ray detector is set for the acquisition of the CT measurement data from the first body region of the patient than for the acquisition of the CT measurement data from the second body region of the patient.

43. Method according to one of the preceding claims, wherein the interface acquires further measurement data which is acquired by means of an imaging device of a radiotherapy device used for the irradiation of the patient, wherein the result data is set in relation to the further measurement data for the planning of the irradiation of the patient.

44. Method according to claim 43, wherein the imaging device of the radiotherapy device used for the irradiation of the patient likewise has a quantum counting X-ray detector, wherein the result data is set in relation to the further measurement data using a specific information content of the CT measurement data and the further CT measurement data resulting from the use of the quantum counting X-ray detector in the acquisition of the CT measurement data and the further measurement data.

45. Method according to one of the preceding claims, wherein the CT measurement data comprises at least two CT measurement data sets which have been acquired at different times, wherein the at least two CT measurement data sets are registered using the specific information content of the CT measurement data resulting from the use of the at least one quantum counting X-ray detector in the acquisition of the CT measurement data, wherein the registered at least two CT measurement data sets are made available to the interface.

46. Method according to one of the preceding claims, wherein a contrast medium has been used during the acquisition of the CT measurement data, wherein the processing of the CT measurement data comprises a material decomposition of the CT measurement data in tissue having contrast media, and other tissue using the specific information content of the CT measurement data resulting from the use of the at least one quantum counting X-ray detector in the acquisition of the CT measurement data.

47. Method according to one of the preceding claims, wherein a contrast medium has been used during the acquisition of the CT measurement data, wherein the processing of the CT measurement data comprises the creation of a virtual non-contrasted CT image using the specific information content of the CT measurement data resulting from the use of the at least one quantum counting X-ray detector in the acquisition of the CT measurement data.

48. Arithmetic unit comprising at least one calculation module, wherein the arithmetic unit is designed to execute a method according to one of the preceding claims.

49. CT device with an arithmetic unit according to claim 48.

50. Computer program product which can be loaded directly into a memory of a programmable arithmetic unit, with program code resources to execute a method according to one of claims 1-47 when the computer program product is executed in the arithmetic unit.

## Revendications

1. Procédé pour disposer de données de résultat, qui sont propres à une utilisation dans une planification d'une exposition d'un patient à du rayonnement, comprenant les stades de procédé suivants :
- on saisit des données de mesure CT du patient, qui ont été acquises au moyen d'un appareil CT, qui a un détecteur de rayons X comptant les quantas,
- on traite ultérieurement les données de mesure CT, dans lequel, lors du traitement ultérieur du traitement de données de mesure CT, on prend en compte un contenu informationnel spécifique des données de mesure CT, qui provient de l'utilisation du détecteur de rayons X comptant les quantas, lors de l'acquisition des données de mesure CT, dans lequel, lors du traitement ultérieur des données de mesure CT, on produit des données de résultat, qui sont propres à être utilisées dans la planification de l'exposition du patient à du rayonnement,
- on met les données de résultat à disposition d'une interface, de manière à pouvoir utiliser les données de résultat pour la planification de l'exposition du patient à du rayonnement.

2. Procédé suivant la revendication 1, dans lequel le procédé comprend le stade de procédé supplémentaire suivant :
- on effectue la planification de l'exposition du patient à du rayonnement, dans lequel on appelle les données de résultat de l'interface et on les utilise lors de la planification de l'exposition du patient à du rayonnement.

3. Procédé suivant l'une des revendications précédentes, dans lequel
- la saisie des données de mesure CT comprend une saisie de données de mesure CT à résolution spectrale, qui proviennent de l'acquisition au moyen du détecteur de rayons X comptant les quantas,
- le traitement ultérieur des données de mesure CT comprend un calcul d'une distribution à résolution spatiale d'un coefficient quantitatif de matériau, à partir des données de mesure CT à résolution spectrale, et
- la fourniture des données de résultat comprend une mise de la distribution à résolution spatiale du coefficient quantitatif de matériau à disposition de l'interface.

4. Procédé suivant la revendication 3, dans lequel le traitement ultérieur des données de mesure CT comprend un calcul d'une première distribution à résolution spatiale d'un premier coefficient quantitatif de matériau et une deuxième distribution à résolution spatiale d'un deuxième coefficient quantitatif de matériau, à partir des données de mesure CT à résolution spectrale.

5. Procédé suivant la revendication 4, dans lequel la fourniture des données de résultat comprend une mise à disposition de la première distribution à résolution spatiale du premier coefficient quantitatif de matériau pour un premier algorithme de calcul de dose et une mise à disposition de la deuxième distribution à résolution spatiale du deuxième coefficient quantitatif de matériau pour un deuxième algorithme de calcul de dose.

6. Procédé suivant l'une des revendications 4 à 5, dans lequel le premier coefficient quantitatif de matériau est une densité électronique et le deuxième coefficient quantitatif de matériau est un numéro atomique effectif.

7. Procédé suivant l'une des revendications 4 à 5, dans lequel le premier coefficient quantitatif de matériau et le deuxième coefficient quantitatif de matériau reposent sur des matériaux de base indépendants linéairement l'un de l'autre.

8. Procédé suivant l'une des revendications 4 à 7, dans lequel le traitement ultérieur des données de mesure CT comprend un traitement ultérieur distinct de la première distribution à résolution spatiale du premier coefficient quantitatif de matériau et de la deuxième distribution à résolution spatiale du deuxième coefficient quantitatif de matériau et une production des données de résultat au moyen d'une réunion des données de résultat partiel produits dans le traitement ultérieur distinct.

9. Procédé suivant l'une des revendications 3 à 8, dans lequel la saisie des données de mesure CT comprend une charge de données de mesure CT à résolution spatiale acquises auparavant à partir d'une base de données pour le calcul de la distribution à résolution spatiale du coefficient quantitatif de matériau.

10. Procédé suivant l'une des revendications 3 à 9, dans lequel le traitement ultérieur des données de mesure CT comprend, en outre, pour le calcul de la distribution à résolution spatiale du coefficient quantitatif de matériau à partir des données de mesure CT à résolution spectrale, une identification d'un volume cible et/ou d'un volume à risque dans les données de mesure CT à résolution spectrale.

11. Procédé suivant la revendication 10, dans lequel on a acquis les données de mesure CT à résolution spectrale au moyen de paramètres d'enregistrement du détecteur à rayons X comptant les quantas, en ce que les données de mesure CT à résolution spectrale ont, pour l'identification du volume cible et/ou du volume à risque, un contraste particulièrement approprié entre divers types de tissus.

12. Procédé suivant l'une des revendications 3 à 11, dans lequel les données de mesure CT à résolution spectrale recouvrent un champ de mesure, qui comprend, dans une couche de mesure axiale, tant tout le corps du patient qu'également les auxiliaires de mise en position pour coucher le patient.

13. Procédé suivant l'une des revendications 3 à 12, dans lequel le traitement ultérieur des données de mesure CT comprend une correction de dureté du rayonnement des données de mesure CT à résolution spectrale, en utilisant des informations spectrales contenues dans les données de mesure CT à résolution spectrale, dans lequel de la distribution à résolution spatiale du coefficient quantitatif de matériau s'effectue à partir des données de mesure CT à résolution spectrale corrigées par la correction de dureté du rayonnement.

14. Procédé suivant l'une des revendications précédentes, dans lequel le traitement ultérieur des données de mesure CT comprend une identification de volume cible et/ou de volume à risque dans les données de mesure CT, dans lequel la mise des données de résultat à disposition de l'interface comprend une mise à disposition du volume cible et/ou du volume à risque identifié.

15. Procédé suivant la revendication 14, dans lequel l'identification du volume cible et/ou du volume à risque comprend une délimitation d'un tissu du volume cible et/ou du volume à risque par rapport à des tissus environnants, la délimitation du tissu du volume cible et/ou du volume à risque par rapport aux tissus environnants a lieu en utilisant le contenu informationnel spécifique des données de mesure CT, qui provient de l'utilisation du au moins un détecteur à rayons X comptant les quantas, lors de l'acquisition des données de mesure CT.

16. Procédé suivant l'une des revendications 14 à 15, dans lequel l'identification du volume cible et/ou du volume à risque comprend une délimitation de tissu adipeux et de tissu musculaire, dans lequel la délimitation du tissu adipeux et du tissu musculaire a lieu en utilisant le contenu informationnel spécifique des données de mesure CT, qui provient de l'utilisation du au moins un détecteur de rayons X comptant les quantas lors de l'acquisition des données de mesure CT.

17. Procédé suivant l'une des revendications 14 à 16, dans lequel l'identification du volume cible et/ou du volume à risque comprend une délimitation de substance grise du cerveau et de substance blanche du cerveau, dans lequel la délimitation de la substance grise du cerveau et de la substance blanche du cerveau a lieu en utilisant le contenu informationnel spécifique des données de mesure CT, qui provient de l'utilisation du au moins un détecteur à rayons X comptant les quantas lors de l'acquisition des données de mesure CT.

18. Procédé suivant l'une des revendications 14 à 17, dans lequel
- la saisie des données de mesure CT comprend une saisie de données de mesure CT à résolution spectrale, qui provient de l'acquisition au moyen du détecteur à rayons X comptant les quantas,
- le traitement ultérieur des données de mesure CT comprend une correction de dureté de rayonnement des données de mesure CT à résolution spectrale en utilisant des informations spectrales contenues dans les données de mesure CT à résolution spectrale, et
- l'identification du volume cible et/ou du volume à risque comprend une délimitation de structures osseuses dans les données de mesure CT à résolution spectrale corrigées par la correction de dureté du rayonnement.

19. Procédé suivant la revendication 18, dans lequel on délimite entre elles les structures osseuses dans une région de base du crâne du patient.

20. Procédé suivant l'une des revendications 14 à 19, dans lequel
- les données de mesure CT comprennent un premier ensemble de données de mesure CT et un deuxième ensemble de données de mesure CT, dans lequel le premier ensemble de données de mesure CT a été acquis au moyen d'un seuil d'énergie plus haut du détecteur à rayons X comptant les quantas que le deuxième ensemble de données de mesure CT,
- le traitement ultérieur des données de mesure CT comprend un établissement d'une combinaison pondérée du premier ensemble de données de mesure CT et du deuxième ensemble de données de mesure CT, des données de mesure CT combinées étant produites, et
- l'identification du volume cible et/ou du volume à risque a lieu dans les données de mesure CT combinées.

21. Procédé suivant la revendication 20, dans lequel
- le traitement ultérieur des données de mesure CT comprend une extraction d'informations pour une correction de dureté de rayonnement à partir du premier jeu de données de mesure CT, et une correction de dureté de rayonnement des données de mesure CT combinées sur la base des informations extraites, dans lequel
- l'identification du volume cible et/ou du volume à risque a lieu dans les données de mesure CT combinées corrigées par la correction de dureté du rayonnement.

22. Procédé suivant l'une des revendications 20 à 21, dans lequel l'établissement de la combinaison pondérée du premier ensemble de données de mesure CT et du deuxième ensemble de données de mesure CT a lieu en utilisant des facteurs de pondération variant dans l'espace.

23. Procédé suivant la revendication 22, dans lequel
- le traitement ultérieur des données de mesure CT comprend d'abord une identification grossière du volume cible et/ou du volume à risque, dans lequel on définit les facteurs de pondération variant dans l'espace, à l'aide du volume cible et/ou du volume à risque segmenté grossièrement et a lieu ensuite une identification fine du volume cible et/ou du volume à risque dans les données de mesure CT combinées.

24. Procédé suivant l'une des revendications précédentes, dans lequel la saisie des données de mesure CT comprend une acquisition de données de mesure CT à haute résolution au moyen du détecteur à rayons X comptant les quantas, dans lequel on utilise un mode à haute résolution du détecteur à rayons X comptant les quantas pour l'acquisition des données de mesure CT à haute résolution, dans lequel on compte séparément dans le mode à haute résolution chaque pixel du détecteur à rayons X comptant les quantas.

25. Procédé suivant la revendication 24, dans lequel
- le traitement ultérieur des données de mesure CT comprend un calcul d'une distribution à résolution spatiale d'un coefficient quantitatif de matériau à partir des données de mesure CT à haute résolution, et
- la mise à disposition des données de résultat comprend une mise de la distribution à résolution spatiale du coefficient quantitatif de matériau à disposition de l'interface.

26. Procédé suivant la revendication 25, dans lequel
- les données de mesure CT à haute résolution entrent, sous une forme inchangée, dans le calcul de la distribution à résolution spatiale du coefficient quantitatif de matériau pour un tissu osseux du patient, et
- les données de mesure CT à haute résolution entrent, suivant une résolution réduite, dans le calcul de la distribution à résolution spatiale du coefficient quantitatif de matériau pour un tissu de partie molle du patient.

27. Procédé suivant la revendication 26, dans lequel on produit la résolution réduite des données de mesure CT à haute résolution au moyen d'un filtrage des données de mesure CT à haute résolution en utilisant un noyau de filtre.

28. Procédé suivant l'une des revendications 25 à 27, dans lequel
- le calcul de la distribution à résolution spatiale du coefficient quantitatif de matériau comprend un calcul d'une distribution à résolution spatiale d'un pouvoir de ralentissement à partir des données de mesure CT à haute résolution, et
- la mise à disposition des données de résultat comprend une mise à disposition de la distribution à résolution spatiale du pouvoir de ralentissement pour une planification de l'exposition du patient à des particules.

29. Procédé suivant l'une des revendications 25 à 28, dans lequel le calcul de la distribution à résolution spatiale du coefficient quantitatif de matériau à partir des données de mesure CT à haute résolution a lieu pour au moins l'une des régions du corps suivante du patient :
- une région de l'œil du patient,
- une région du corps décrite par un tracé du nerf du trijumeau du patient,
- une base du crâne du patient,
- une région des poumons du patient.

30. Procédé suivant l'une des revendications précédentes, dans lequel
- la saisie des données de mesure CT comprend une saisie de données de mesure CT à résolution spectrale et à résolution temporelle, qui proviennent de l'acquisition au moyen du détecteur à rayons X comptant les quantas,
- le traitement ultérieur des données de mesure CT comprend un calcul d'une distribution à résolution spatiale et à résolution temporelle d'un coefficient quantitatif de matériau à partir des données de mesure CT à résolution spectrale et à résolution temporelle, et
- la mise à disposition des données de résultat comprend une mise de la distribution à résolution spatiale et à résolution temporelle du coefficient quantitatif de matériau à disposition de l'interface.

31. Procédé suivant la revendication 30, dans lequel on saisit les données de mesure CT à résolution spectrale et à résolution temporelle à partir d'au moins l'une des régions du corps suivante du patient :
- une région du foie du patient,
- une région du pancréas du patient,
- une région du thorax du patient.

32. Procédé suivant l'une des revendications précédentes, dans lequel la saisie des données de mesure CT du patient comprend l'acquisition des données de mesure CT au moyen de l'appareil CT, qui a le détecteur de rayons X comptant les quantas, dans lequel, pendant l'acquisition des données de mesure CT, on modifie un paramètre du détecteur de rayons X comptant les quantas.

33. Procédé suivant la revendication 32, dans lequel on modifie, pendant l'acquisition des données de mesure CT, un seuil d'énergie du détecteur à rayons X comptant les quantas.

34. Procédé suivant l'une des revendications 32 à 33, dans lequel, pendant l'acquisition des données de mesure CT, on modifie une résolution du détecteur de rayons X comptant les quantas, en ce qui concerne une combinaison de pixels du détecteur à rayons X comptant les quantas.

35. Procédé suivant l'une des revendications 32 à 34, dans lequel on modifie le paramètre du détecteur de rayons X comptant les quantas pendant l'acquisition des données de mesure CT, de manière à mettre, pendant une acquisition des données de mesure CT à une première position z le long d'une direction longitudinale du patient, un premier réglage du paramètre du détecteur, et pendant une acquisition des données de mesure CT à une deuxième position z le long de la direction longitudinale du patient, un deuxième réglage du paramètre du détecteur.

36. Procédé suivant l'une des revendications 32 à 35, dans lequel on modifie le paramètre du détecteur de rayons X comptant les quantas pendant l'acquisition des données de mesure CT, de manière à mettre, pendant une acquisition des données de mesure CT pour une première position en rotation d'un système de détecteur - émetteur de l'appareil CT, un premier réglage du paramètre du détecteur, et pendant une acquisition des données de mesure CT à une deuxième position en rotation du système détecteur - émetteur de l'appareil CT, un deuxième réglage du paramètre du détecteur.

37. Procédé suivant l'une des revendications 32 à 36 précédentes, dans lequel on modifie le paramètre du détecteur à rayons X comptant les quantas, pendant l'acquisition des données de mesure CT, de manière à mettre, pendant une acquisition des données de mesure CT d'une première région du corps du patient, un premier réglage du paramètre du détecteur, et pendant une acquisition des données de mesure CT d'une deuxième région du corps du patient, un deuxième réglage du paramètre du détecteur.

38. Procédé suivant la revendication 37, dans lequel on fixe la première région du corps et la deuxième région du corps avant l'acquisition des données de mesure CT, à l'aide de données d'images médicales déjà présentent du patient et/ou en utilisant une base de données formant atlas.

39. Procédé suivant l'une des revendications 37 à 38, dans lequel la première région du corps comprend un volume cible à exposer au rayonnement et la deuxième région du corps comprend une région du corps se trouvant à l'extérieur du volume cible à exposer au rayonnement.

40. Procédé suivant l'une des revendications 37 à 39, dans lequel la première région du corps est constituée de manière à être placée, lors de l'exposition du patient à du rayonnement, dans une zone de rayonnement, et la deuxième région du corps est constituée de manière à être placée, lors de l'exposition du patient à du rayonnement, en dehors d'une zone de rayonnement.

41. Procédé suivant l'une des revendications 37 à 40, dans lequel la première région du corps et la deuxième région du corps sont constitués de manière à ce que, lors de l'exposition du patient à du rayonnement, il soit prévu une dose de rayonnement plus grande pour la première région du corps que pour la deuxième région du corps.

42. Procédé suivant l'une des revendications 37 à 41, dans lequel, pendant l'acquisition des données de mesure CT, on modifie le paramètre du détecteur de rayons X comptant les quantas, de manière à mettre, pendant l'acquisition des données de mesure CT à partir de la première région du corps du patient, une résolution plus haute du détecteur de rayons X comptant les quantas, que pour l'acquisition des données de mesure CT à partir de la deuxième région du corps du patient.

43. Procédé suivant l'une des revendications précédentes, dans lequel l'interface saisit d'autres données de mesure que l'on saisit au moyen d'une installation d'imagerie d'un appareil de radiothérapie utilisée pour l'exposition du patient à du rayonnement, dans lequel, pour la planification de l'exposition du patient à du rayonnement, on règle les données du résultat par rapport aux autres données de mesure.

44. Procédé suivant la revendication 43, dans lequel l'installation d'imagerie de l'appareil de radiothérapie utilisée pour l'exposition du patient à du rayonnement a également un détecteur de rayons X comptant les quantas, dans lequel on règle, par rapport aux autres données de mesure, les données de résultat en utilisant un contenu informationnel spécifique des données de mesure CT et des autres données de mesure CT, qui proviennent de l'utilisation du détecteur à rayons X comptant les quantas lors de l'acquisition des données de mesure CT et des autres données de mesure CT.

45. Procédé suivant l'une des revendications précédentes, dans lequel les données de mesure CT comprennent au moins deux ensembles de données de mesure CT, qui ont été saisies à des instants différents, dans lequel on enregistre les au moins deux ensembles de données de mesure CT, en utilisant le contenu informationnel spécifique des données de mesure CT, qui provient de l'utilisation du au moins un détecteur à rayons X comptant les quantas lors de l'acquisition des données de mesure CT, dans lequel on met à disposition de l'interface les au moins deux ensembles de données de mesure CT enregistrés.

46. Procédé suivant l'une des revendications précédentes, dans lequel on a utilisé un agent de contraste pendant l'acquisition des données de mesure CT, dans lequel le traitement ultérieur des données de mesure CT comprend une décomposition de matière des données de mesure CT dans du tissu, qui a l'agent de contraste, et dans le reste du tissu en utilisant le contenu informationnel spécifique des données de mesure CT, qui provient de l'utilisation, lors de l'acquisition des données de mesure CT, du au moins un détecteur de rayons X comptant les quantas.

47. Procédé suivant l'une des revendications précédentes, dans lequel on a utilisé, pendant l'acquisition des données de mesure CT, un agent de contraste, dans lequel le traitement ultérieur des données de mesure CT comprend une production d'une image CT virtuelle non contrastée en utilisant le contenu informationnel spécifique des données de mesure CT, qui provient de l'utilisation, lors de l'acquisition des données de mesure CT, du au moins un détecteur de rayons X comptant les quantas.

48. Unité informatique comprenant au moins un module de calcul, dans lequel l'unité informatique est constituée pour effectuer un procédé suivant l'une des revendications précédentes.

49. Appareil CT comprenant une unité informatique suivant la revendication 48.

50. Produit de programme d'ordinateur, qui peut être chargé directement dans une mémoire d'une unité informatique programmable, comprenant des moyens de code de programme pour effectuer un procédé suivant l'une des revendications 1 à 47, lorsque le produit de programme d'ordinateur est réalisé dans l'unité informatique.
